# EUROPEAN PATENT APPLICATION

(11) **EP 4 321 537 A1**
(43) Date of publication of application: **14.02.2024**
(21) Application number: 23745372.5
(22) Date of filing: 13.04.2023
(51) Int. Cl.: C07K 16/46, C12N 15/13, C12N 5/20, C07K 16/28, C07K 16/40, A61K 39/395, A61P 35/00, A61P 7/06

(54) **PHARMACEUTICAL COMPOSITION AND USE THEREOF**

(30) Priority: 22.06.2022 CN 202210712836
(71) Applicant: Akeso Biopharma, Inc., Zhongshan, Guangdong 528437 (CN)
(72) Inventor: LI, Baiyong, Guangdong 528437 (CN); XIA, Yu, Guangdong 528437 (CN); WANG, Zhongmin, Guangdong 528437 (CN)
(74) Representative: Cooley (UK) LLP
(86) International application number: PCT/CN2023/087998
(87) International publication number: WO 2023/246247

(57) **Abstract**

The present invention relates to a pharmaceutical composition, which comprises an anti-CD73 (e.g., human CD73) antibody or an antigen-binding fragment thereof, and an anti-PD-1-anti-VEGFA bispecific antibody or an antigen-binding fragment thereof. Specifically, a heavy chain variable region of the anti-CD73 antibody comprises HCDR1-HCDR3 having amino acid sequences set forth in SEQ ID NOs: 15-17; and a light chain variable region of the antibody comprises LCDR1-LCDR3 having amino acid sequences set forth in SEQ ID NOs: 18-20.

## Description

### TECHNICAL FIELD

The present invention relates to the field of immunology, and specifically relates to a pharmaceutical composition, which comprises an anti-CD73 (e.g., human CD73) antibody or an antigen-binding fragment thereof, and an anti-PD-1-anti-VEGFA bispecific antibody or an antigen-binding fragment thereof.

### BACKGROUND

Ecto-5'-nucleotidase, namely CD73 protein, is a multifunctional glycoprotein encoded by NT5E gene and having a molecular weight of 70 KD, which is anchored on a cell membrane by glycosyl phosphatidy linositol (GPI) (Zimmermann H., Biochem J., 1992; 285:345-365).

CD73 is widely distributed on the surface of human tissue cells, and it has been found in research that CD73 is highly expressed in various solid tumors, specifically in cancer cells, dendritic cells, regulatory T cells (Tregs), natural killer cells (NK cells), myeloid-derived suppressor cells (MDSCs), tumor-associated macrophages (TAMs) and the like in a tumor micro environment. Hypoxia induces the up-regulation of molecules such as hypoxia-inducible factor-1 (HIF-1), thereby leading to the widespread expression of CD73 in the tumor micro environment (Synnestvedt K, et al. J Clin Invest. 2002; 110:993-1002). Analysis of clinical tumor samples has shown that high expression of CD73 is a potential biomarker and is closely related to adverse prognosis of various types of tumors, including breast cancer, lung cancer, ovarian cancer, kidney cancer, gastric cancer, head and neck cancer and the like.

CD73 has both hydrolase activity and non-hydrolase activity. The enzyme and non-enzyme functions of CD73 simultaneously work in the related process in tumors, and mutually promote and maintain the progression of tumors. More and more studies have found that CD73 is a key regulatory molecule for tumor cell proliferation, metastasis and invasion *in vitro,* and tumor angiogenesis and tumor immune escape mechanism *in vivo,* wherein an important immune suppression mechanism is mediated by CD73-adenosine metabolic signaling pathway. CD39 at the upstream of CD73 can catalyze ATP to generate adenosine monophosphate (AMP), the generated AMP is converted into adenosine by CD73, and adenosine binds to a downstream adenosine receptor (A2AR). A2AR inhibits a series of signaling pathways related to immune activation, such as LCK, MAPK, and PKC, and inhibits the immune killing effect of T cells by activating protein kinase A (PKA) and Csk kinase, thereby playing an immune suppression role (Antonioli L, et al. Nat Rev Cancer. 2013; 13:842-857).

The transmembrane receptor PD-1 (programmed cell death protein-1) is a member of the CD28 family, and is expressed in activated T cells, B cells, and myeloid cells. Ligands of PD-1, both PDL1 (programmed cell death 1 ligand 1, or PDL-1) and PDL2 (programmed cell death 1 ligand 2, or PDL-2), are members of the B7 superfamily. PDL1 is expressed in a variety of cells including T cells, B cells, endothelial cells and epithelial cells, and PDL2 is expressed only in antigen presenting cells such as dendritic cells and macrophages.

The PD-1/PDL1 signaling pathway plays an important role in regulating immune tolerance, microbial infection, and tumor immune escape. PD-1 is mainly expressed in immune cells such as T cells, and the ligand PDL1 of PD-1 is highly expressed in a plurality of human tumor tissues. Blocking the PD-1/PDL1 signaling pathway may activate inhibited T cells, which thus attack cancer cells. Blocking the PD-1/PDL1 signaling can promote the proliferation of tumor antigen-specific T cells, activate the tumor cell killing process, and further inhibit local tumor growth (Julie R et al., 2012, N Engl J Med., 366:2455-2465). In addition, tumors with high PDL1 expression are associated with cancers that are difficult to detect (Hamanishi et al., 2007, Proc. Natl. Acad. Sci. USA, 104:3360-5). An effective method is administering an anti-PD-1 antibody to modulate the expression of PD-1. Due to the broad anti-tumor prospects and surprising efficacy of PD-1 antibodies, it is widely accepted in the industry that antibodies targeting the PD-1 pathway will bring about breakthroughs in various tumors, for example, non-small cell lung cancer, renal cell carcinoma, ovarian cancer, melanoma (Homet M. B., Parisi G., et al., 2015, Semin Oncol., 42(3):466-473), leukemia and anemia (Held SA, Heine A, et al., 2013, Curr Cancer Drug Targets., 13(7):768-74).

Vascular endothelial growth factor (VEGF) is a growth factor that can promote the division and proliferation of endothelial cells, promote the formation of new blood vessels and improve blood vessel permeability. It binds to vascular endothelial growth factor receptors on the cell surface and plays a role by activating tyrosine kinase signal transduction pathways. In tumor tissues, tumor cells, and macrophages and mast cells invading into tumors can secrete high-level VEGF, stimulate tumor vascular endothelial cells in a paracrine form, promote proliferation and migration of endothelial cells, induce angiogenesis, promote continuous growth of tumor, improve vascular permeability, cause fibrin deposition in surrounding tissues, and promote infiltration of mononuclear cells, fibroblasts and endothelial cells, which facilitates formation of tumor stroma and entry of tumor cells into new blood vessels, and promote tumor metastasis. Therefore, inhibiting tumor angiogenesis is considered to be one of the most promising tumor treatment methods at present. The VEGF family includes VEGFA, VEGFB, VEGFC, VEGFD, and PIGF. Vascular Endothelial Growth Factor Receptors (VEGFRs) include VEGFR1 (also known as Flt1), VEGFR2 (also known as KDR or Flk1), VEGFR3 (also known as Flt4), and Neuropilin-1 (NRP-1), wherein the first three receptors are similar in structure, belong to a tyrosine kinase superfamily, and are composed of an extramembrane region, a transmembrane segment and an intramembrane region, where the extramembrane region is composed of an immunoglobulin-like domain, and the intramembrane region is a tyrosine kinase region. VEGFR1 and VEGFR2 are mainly found on the surface of vascular endothelial cells, and VEGFR3 is mainly found on the surface of lymphatic endothelial cells.

Molecules of the VEGF family have different affinities for these receptors.

VEGFA mainly acts in combination with VEGFR1, VEGFR2 and NRP-1. VEGFR1 is the first recognized receptor, and has a higher affinity for VEGFA than VEGFR2 under normal physiological conditions but a lower tyrosinase activity in the intracellular segment than VEGFR2 (Ma Li, Chinese Journal of Birth Health and Heredity, 24(5) (2016): 146-148).

VEGFR2 is the primary regulator of angiogenesis and vascular engineering, and has a much higher tyrosine kinase activity than VEGFR1. VEGFR2, after binding to ligand VEGFA, mediates the proliferation, differentiation and the like of vascular endothelial cells, as well as the formation process of blood vessels and the permeability of blood vessels (Roskoski R Jr. et al., Crit Rev Oncol Hematol, 62(3) (2007):179-213). VEGFA, after binding to VEGFR2, mediates the transcriptional expression of intracellular related protein genes through the downstream PLC-γ-PKC-Raf-MEK-MAPK signaling pathway, and thus promotes the proliferation of vascular endothelial cells (Takahashi T et al., Oncogene, 18(13) (1999):2221-2230).

VEGFR3 is a member of the tyrosine kinase family, and mainly expressed in embryonic vascular endothelial cells and adult lymphatic endothelial cells, and VEGFC and VEGFD bind to VEGFR3 to stimulate proliferation and migration of lymphatic endothelial cells and promote neogenesis of lymphatic vessels; NRP-1 is a non-tyrosine kinase transmembrane protein and is incapable of independently transducing biological signals, and it is able to mediate signaling only after forming a complex with a VEGF tyrosine kinase receptor (Ma Li, Chinese Journal of Birth Health and Heredity, 24(5) (2016): 146-148). VEGFA and VEGFR2 are mainly involved in regulation of angiogenesis, where before and after the binding of VEGFA to VEGFR2, a cascade reaction of numerous intermediate signals in upstream and downstream pathways is formed, and finally the physiological functions are changed by proliferation, survival, migration, permeability increase, infiltration to peripheral tissues and other patterns of endothelial cells (Dong Hongchao et al., Sep. 2014, Journal of Modern Oncology, 22(9):2231-3).

Currently, there are several humanized monoclonal antibodies targeting human VEGF, particularly VEGFA, such as bevacizumab, which has been approved by the U.S. Food and Drug Administration for the treatment of various tumors such as non-small cell lung cancer, renal cell carcinoma, cervical cancer, and metastatic colorectal cancer in succession during 2004.

In summary, developing a treatment or combination therapy with higher efficacy is of great meaning.

### SUMMARY

After intensive studies and creative efforts, the inventors used mammalian cell expression systems to express recombinant human CD73 as an antigen to immunize mice, and obtained hybridoma cells by fusion of mouse spleen cells and myeloma cells. The inventor obtained a hybridoma cell line LT014 (accession number: CCTCC NO: C2018137) by screening a large number of samples.

The inventors have surprisingly found that the hybridoma cell line LT014 can secrete a specific monoclonal antibody (named as 19F3) specifically binding to human CD73, and the monoclonal antibody can effectively inhibit the enzyme activity reaction of CD73 in a non-substrate competition mode, reduce the production of adenosine, promote the activity of T cells, and exert the effect of inhibiting tumor growth.

Further, the inventors have creatively prepared a humanized antibody against human CD73 (named as 19F3H1L1(hG1DM), 19F3H2L2(hG1DM), 19F3H2L3 and 19F3H2L3(hG1DM)), and further, the anti-CD73 antibody has introduced amino acid mutations to eliminate ADCC and CDC effects, avoiding undesired toxicity mediated by the antibody.

The inventors have also surprisingly found that the antibody in combination with the anti-PD-1/VEGFA bispecific antibody of the present invention has a pharmacological effect of effectively inhibiting tumor growth, which is superior to that of either the anti-PD-1/VEGFA bispecific antibody or the anti-CD73 antibody alone.

Another aspect of the present invention further relates to an antibody, wherein the anti-CD73 antibody comprises:
HCDR1, HCDR2 and HCDR3 of a heavy chain variable region set forth in SEQ ID NO: 2, SEQ ID NO: 6 or SEQ ID NO: 10; and LCDR1, LCDR2 and LCDR3 of a light chain variable region set forth in SEQ ID NO: 4, SEQ ID NO: 8, SEQ ID NO: 12 or SEQ ID NO: 14;
preferably, according to an IMGT numbering system, the anti-CD73 antibody comprises:
HCDR1 comprising or consisting of an amino acid sequence set forth in SEQ ID NO: 15, a sequence having at least 80%, at least 81%, at least 82%, at least 83%, at least 84%, at least 85%, at least 86%, at least 87%, at least 88%, at least 89%, at least 90%, preferably at least 91%, at least 92%, at least 93%, at least 94%, at least 95%, at least 96%, at least 97%, at least 98% or at least 99% sequence identity to the sequence, or an amino acid sequence having one or more (preferably 1, 2 or 3) conservative amino acid mutations (preferably substitutions, insertions or deletions) compared to the sequence,
HCDR2 comprising or consisting of an amino acid sequence set forth in SEQ ID NO: 16, a sequence having at least 80%, at least 81%, at least 82%, at least 83%, at least 84%, at least 85%, at least 86%, at least 87%, at least 88%, at least 89%, at least 90%, preferably at least 91%, at least 92%, at least 93%, at least 94%, at least 95%, at least 96%, at least 97%, at least 98% or at least 99% sequence identity to the sequence, or an amino acid sequence having one or more (preferably 1, 2 or 3) conservative amino acid mutations (preferably substitutions, insertions or deletions) compared to the sequence,
HCDR3 comprising or consisting of an amino acid sequence set forth in SEQ ID NO: 17, a sequence having at least 80%, at least 81%, at least 82%, at least 83%, at least 84%, at least 85%, at least 86%, at least 87%, at least 88%, at least 89%, at least 90%, preferably at least 91%, at least 92%, at least 93%, at least 94%, at least 95%, at least 96%, at least 97%, at least 98% or at least 99% sequence identity to the sequence, or an amino acid sequence having one or more (preferably 1, 2 or 3) conservative amino acid mutations (preferably substitutions, insertions or deletions) compared to the sequence,
LCDR1 comprising or consisting of an amino acid sequence set forth in SEQ ID NO: 18, a sequence having at least 80%, at least 81%, at least 82%, at least 83%, at least 84%, at least 85%, at least 86%, at least 87%, at least 88%, at least 89%, at least 90%, preferably at least 91%, at least 92%, at least 93%, at least 94%, at least 95%, at least 96%, at least 97%, at least 98% or at least 99% sequence identity to the sequence, or an amino acid sequence having one or more (preferably 1, 2 or 3) conservative amino acid mutations (preferably substitutions, insertions or deletions) compared to the sequence,
LCDR2 comprising or consisting of an amino acid sequence set forth in SEQ ID NO: 19, a sequence having at least 80%, at least 81%, at least 82%, at least 83%, at least 84%, at least 85%, at least 86%, at least 87%, at least 88%, at least 89%, at least 90%, preferably at least 91%, at least 92%, at least 93%, at least 94%, at least 95%, at least 96%, at least 97%, at least 98% or at least 99% sequence identity to the sequence, or an amino acid sequence having one or more (preferably 1, 2 or 3) conservative amino acid mutations (preferably substitutions, insertions or deletions) compared to the sequence, and
LCDR3 comprising or consisting of an amino acid sequence set forth in SEQ ID NO: 20, a sequence having at least 80%, at least 81%, at least 82%, at least 83%, at least 84%, at least 85%, at least 86%, at least 87%, at least 88%, at least 89%, at least 90%, preferably at least 91%, at least 92%, at least 93%, at least 94%, at least 95%, at least 96%, at least 97%, at least 98% or at least 99% sequence identity to the sequence, or an amino acid sequence having one or more (preferably 1, 2 or 3) conservative amino acid mutations (preferably substitutions, insertions or deletions) compared to the sequence.

In some embodiments of the present invention,
the heavy chain variable region of the antibody comprises or consists of the following sequences:
   SEQ ID NO: 2, SEQ ID NO: 6 or SEQ ID NO: 10, a sequence having at least 80%, at least 81%, at least 82%, at least 83%, at least 84%, at least 85%, at least 86%, at least 87%, at least 88%, at least 89%, at least 90%, preferably at least 91%, at least 92%, at least 93%, at least 94%, at least 95%, at least 96%, at least 97%, at least 98% or at least 99% sequence identity to SEQ ID NO: 2, SEQ ID NO: 6 or SEQ ID NO: 10, or an amino acid sequence having one or more (preferably 1, 2, 3, 4, 5, 6, 7, 8, 9 or 10) conservative amino acid mutations (preferably substitutions, insertions or deletions) compared to the amino acid sequences set forth in SEQ ID NO: 2, SEQ ID NO: 6 or SEQ ID NO: 10, and
the light chain variable region of the antibody comprises or consists of the following sequences:
   SEQ ID NO: 4, SEQ ID NO: 8, SEQ ID NO: 12 or SEQ ID NO: 14, a sequence having at least 80%, at least 81%, at least 82%, at least 83%, at least 84%, at least 85%, at least 86%, at least 87%, at least 88%, at least 89%, at least 90%, preferably at least 91%, at least 92%, at least 93%, at least 94%, at least 95%, at least 96%, at least 97%, at least 98% or at least 99% sequence identity to SEQ ID NO: 4, SEQ ID NO: 8, SEQ ID NO: 12 or SEQ ID NO: 14, or an amino acid sequence having one or more (preferably 1, 2, 3, 4, 5, 6, 7, 8, 9 or 10) conservative amino acid mutations (preferably substitutions, insertions or deletions) compared to the amino acid sequences set forth in SEQ ID NO: 4, SEQ ID NO: 8, SEQ ID NO: 12 or SEQ ID NO: 14.

In some embodiments of the present invention, an amino acid sequence of the heavy chain variable region of the antibody is set forth in SEQ ID NO: 2 (preferably, a nucleic acid sequence is set forth in SEQ ID NO: 1), and an amino acid sequence of the light chain variable region of the antibody is set forth in SEQ ID NO: 4 (preferably, a nucleic acid sequence is set forth in SEQ ID NO: 3);
an amino acid sequence of the heavy chain variable region of the antibody is set forth in SEQ ID NO: 6 (preferably, a nucleic acid sequence is set forth in SEQ ID NO: 5), and an amino acid sequence of the light chain variable region of the antibody is set forth in SEQ ID NO: 8 (preferably, a nucleic acid sequence is set forth in SEQ ID NO: 7);
an amino acid sequence of the heavy chain variable region of the antibody is set forth in SEQ ID NO: 10 (preferably, a nucleic acid sequence is set forth in SEQ ID NO: 9), and an amino acid sequence of the light chain variable region of the antibody is set forth in SEQ ID NO: 12 (preferably, a nucleic acid sequence is set forth in SEQ ID NO: 11); or
an amino acid sequence of the heavy chain variable region of the antibody is set forth in SEQ ID NO: 10 (preferably, a nucleic acid sequence is set forth in SEQ ID NO: 9), and an amino acid sequence of the light chain variable region of the antibody is set forth in SEQ ID NO: 14 (preferably, a nucleic acid sequence is set forth in SEQ ID NO: 13).

In some embodiments of the present invention, the heavy chain constant region of the antibody is an Ig gamma-1 chain C region, ACCESSION: P01857; and the light chain constant region is an Ig kappa chain C region, ACCESSION: P01834. More preferably, the heavy chain constant region of the anti-CD73 antibody has the following mutations based on the sequence set forth in ACCESSION: P01857 according to the EU numbering system:
L234A and L235A; or
L234A and G237A; or
L235A and G237A;
   or
L234A, L235A and G237A;
or one or more mutations selected from:
N297A, D265A, D270A, P238D, L328E, E233D, H268D, P271G, A330R, C226S, C229S, E233P, P331S, S267E, L328F, A330L, M252Y, S254T, T256E, N297Q, P238S, P238A, A327Q, A327G, P329A, K322A, T394D, G236R, G236A, L328R, A330S, P331S, H268A, E318A and K320A. Most preferably, an amino acid sequence of the heavy chain constant region of the anti-CD73 antibody is set forth in SEQ ID NO: 21, and an amino acid sequence of the light chain constant region of the anti-CD73 antibody is set forth in SEQ ID NO: 22.

In some embodiments of the present invention, the heavy chain constant region of the antibody is an Ig gamma-1 chain C region, ACCESSION: P01857, having a leucine-to-alanine point mutation introduced at position 234 (L234A), and a leucine-to-alanine point mutation introduced at position 235 (L235A), and having an amino acid sequence set forth in SEQ ID NO: 21; the light chain constant region is an Ig kappa chain C region, ACCESSION: P01834, having an amino acid sequence set forth in SEQ ID NO: 22.

The variable regions of the light chain and the heavy chain determine the binding of the antigen; the variable region of each chain contains three hypervariable regions called complementarity determining regions (CDRs) (CDRs of the heavy chain (H) comprise HCDR1, HCDR2 and HCDR3, and CDRs of the light chain (L) comprise LCDR1, LCDR2 and LCDR3, which are named by Kabat et al., see Bethesda M.d., Sequences of Proteins of Immunological Interest, Fifth Edition, NIH Publication 1991; 1-3:91-3242).

Preferably, CDRs may also be defined by the IMGT numbering system, see Ehrenmann, Francois, Quentin Kaas, and Marie-Paule Lefranc., IMGT/3Dstructure-DB and IMGT/DomainGapAlign: a database and a tool for immunoglobulins or antibodies, T cell receptors, MHC, IgSF, and MhcSF. Nucleic acids research, 2009; 38(suppl_1): D301-D307.

The amino acid sequences of the CDRs of the monoclonal antibody sequences are analyzed according to the IMGT definition by technical means well known to those skilled in the art, for example by using the VBASE2 database.

The antibodies 19F3, 19F3H1L1(hG1DM), 19F3H2L2(hG1DM) and 19F3H2L3(hG1DM) involved in the present invention have the same CDRs.

The 3 CDRs of the heavy chain variable region have the following amino acid sequences:
HCDR1: GYSFTGYT (SEQ ID NO: 15),
HCDR2: INPYNAGT (SEQ ID NO: 16), and
HCDR3: ARSEYRYGGDYFDY (SEQ ID NO: 17);
the 3 CDRs of the light chain variable region have the following amino acid sequences:
LCDR1: QSLLNSSNQKNY (SEQ ID NO: 18),
LCDR2: FAS (SEQ ID NO: 19), and
LCDR3: QQHYDTPYT (SEQ ID NO: 20).

In some embodiments of the present invention, the antibody is a monoclonal antibody.

In some embodiments of the present invention, the antibody is a humanized antibody, a chimeric antibody or a multispecific antibody (e.g., a bispecific antibody).

In some embodiments of the present invention, the antigen-binding fragment is selected from Fab, Fab', F(ab')₂, Fd, Fv, dAb, Fab/c, a complementarity determining region fragment, a single chain antibody (e.g., scFv), a humanized antibody, a chimeric antibody and a bispecific antibody.

Yet another aspect of the present invention relates to a conjugate comprising an antibody and a conjugated moiety, wherein the antibody is the antibody or the antigen-binding fragment thereof according to any one of the aspects of the present invention, and the conjugated moiety is a purification tag (e.g., a His tag), a detectable label; preferably, the conjugated moiety is a radioisotope, a fluorescent substance, a chemiluminescent substance, a colored substance, polyethylene glycol or an enzyme.

Yet another aspect of the present invention relates to a fusion protein or a multispecific antibody (preferably a bispecific antibody) comprising the antibody or the antigen-binding fragment thereof according to any one of the aspects of the present invention.

Yet another aspect of the present invention relates to a kit comprising the antibody or the antigen-binding fragment thereof according to any one of the aspects of the present invention, the conjugate, the fusion protein or the multispecific antibody of the present invention; preferably, the kit further comprises a secondary antibody specifically recognizing the antibody; optionally, the secondary antibody further comprises a detectable label, such as a radioisotope, a fluorescent substance, a chemiluminescent substance, a colored substance or an enzyme.

Yet another aspect of the present invention relates to use of the antibody or the antigen-binding fragment thereof according to any one of the aspects of the present invention, the conjugate, the fusion protein or the multispecific antibody of the present invention in preparing a kit used for detecting the presence or level of CD73 in a sample.

Yet another aspect of the present invention relates to a pharmaceutical composition comprising the antibody or the antigen-binding fragment thereof according to any one of the aspects of the present invention, the conjugate, the fusion protein or the multispecific antibody of the present invention; optionally, the pharmaceutical composition further comprises a pharmaceutically acceptable carrier and/or excipient. Preferably, the pharmaceutical composition is in a form suitable for administration by subcutaneous injection, intradermal injection, intravenous injection, intramuscular injection or intralesional injection.

Yet another aspect of the present invention relates to use of the antibody or the antigen-binding fragment thereof according to any one of the aspects of the present invention, the conjugate, the fusion protein or the multispecific antibody of the present invention in preparing a medicament for treating and/or preventing a tumor (such as a solid tumor, preferably non-small cell lung cancer, prostate cancer (including metastatic castration-resistant prostate cancer (mCRPC)), triple-negative breast cancer, ovarian cancer, colorectal cancer (including microsatellite stability (MSS) and mismatch repair dysfunction/microsatellite high instability (dMMR/MSI-H) type), gastric cancer (including microsatellite stability (MSS) and mismatch repair dysfunction/microsatellite high instability (dMMR/MSI-H) type), melanoma, head and neck cancer, renal cell carcinoma or pancreatic ductal adenocarcinoma), or in preparing a medicament for diagnosing a tumor.

Yet another aspect of the present invention relates to a hybridoma cell line LT014, which was deposited at China Center for Type Culture Collection (CCTCC) with the accession number CCTCC NO: C2018137.

Yet another aspect of the present invention relates to a combination product (e.g., a kit) comprising a first product and a second product in separate packages, wherein,
the first product comprises the anti-CD73 antibody or the antigen-binding fragment thereof according to any one of the aspects of the present invention, the conjugate according to the present invention, or the pharmaceutical composition according to any one of the aspects of the present invention;
the second product comprises the anti-PD-1-anti-VEGFA bispecific antibody;
preferably, the combination product further comprises a third product in a separate package comprising one or more chemotherapeutics,
preferably, the first product and the second product further independently comprise one or more pharmaceutically acceptable auxiliary materials;
preferably, the combination product further comprises a product instruction, and preferably, the instruction states that the unit dose of the anti-CD73 antibody and/or the anti-PD-1-anti-VEGFA bispecific antibody is 0.1-100 mg, preferably 1-10 mg per kg body weight; alternatively, the unit dose of the anti-CD73 antibody and/or the anti-PD-1-anti-VEGFA bispecific antibody is 10-1000 mg, preferably 50-500 mg, 100-400 mg, 150-300 mg, 150-250 mg or 200 mg in each subject, and preferably the instruction states that the anti-CD73 antibody and/or the anti-PD-1-anti-VEGFA bispecific antibody is administered twice a day to about once every other day, or once every 3 days, 4 days, 5 days, 6 days, 10 days, 1 week, 2 weeks, 3 weeks, 4 weeks, 5 weeks or 6 weeks.

In one or more embodiments of the present invention, the mass ratio of the anti-CD73 antibody or the antigen-binding fragment thereof to the anti-PD-1-anti-VEGFA bispecific antibody in the kit or pharmaceutical composition is (1:5)-(5:1), e.g., 1:5, 1:4, 1:3, 1:2, 1:1, 2:1, 3:1, 4:1 or 5:1, based on the mass of the antibody.

Yet another aspect of the present invention relates to a method for treating and/or preventing a tumor, comprising administering to a patient a therapeutically effective amount of drug A and a therapeutically effective amount of drug B, wherein the drug A comprises the antibody or the antigen-binding fragment thereof according to the present invention, the conjugate according to the present invention, or the fusion protein or multispecific antibody according to the present invention, and the drug B comprises the anti-PD-1-anti-VEGFA bispecific antibody, preferably the drug A and the drug B are administered either simultaneously or sequentially, wherein the sequential administration is that the drug A is administrated firstly or the drug B is administrated firstly.

In some embodiments of the present invention, a heavy chain amino acid sequence of the anti-PD-1-anti-VEGFA bispecific antibody is set forth in SEQ ID NO: 23 and a light chain amino acid sequence thereof is set forth in SEQ ID NO: 25.

The present invention relates to a method for preventing and/or treating a tumor (especially a malignant tumor), comprising administering to a subject a therapeutically effective amount of the anti-CD73 antibody in combination with the anti-PD-1-anti-VEGFA bispecific antibody, and more preferably further in combination with one or more chemotherapeutic drugs (preferably the chemotherapeutic drug is a chemotherapeutic agent or a growth inhibitor, a targeted therapeutic agent (e.g., an antibody-drug conjugate, an antibody or an antigen-binding fragment thereof), a T cell expressing a chimeric antigen receptor, an angiogenesis inhibitor, an antineoplastic agent, a cancer vaccine, an adjuvant and a combination thereof, an antimetabolite, an antibiotic, a plant-based and/or hormonal drug, preferably cyclophosphamide, pemetrexed, a platinum-based drug such as cisplatin, carboplatin, oxaliplatin, adriamycin, paclitaxel, vinca alkaloid, tamoxifen, megestrol, goserelin, asparaginase and/or a fluorouracil antineoplastic drug), preferably, the anti-CD73 antibody, the anti-PD-1-anti-VEGFA bispecific antibody and the tumor chemotherapeutic drug are administered either simultaneously or sequentially.

In one or more embodiments of the present invention, the chemotherapeutic agent or the growth inhibitor is selected from an alkylating agent, an anthracycline, an anti-hormonal agent (e.g., an anti-androgen agent), an aromatase inhibitor, a protein kinase inhibitor (e.g., a tyrosine kinase inhibitor), a lipid kinase inhibitor, an antisense oligonucleotide, a ribozyme, an anti-metabolite, a topoisomerase inhibitor, a cytotoxic agent or an anti-tumor antibiotic, a proteasome inhibitor, an anti-microtubule agent, an EGFR antagonist, a VEGF antagonist, a PD-1 antagonist, an angiopoietin 2 antagonist, a retinoid, a histone deacetylase inhibitor, and a combination thereof.

In one or more embodiments of the present invention, the targeted therapeutic agent is selected from a B-raf inhibitor, an MEK inhibitor, a K-ras inhibitor, a c-Met inhibitor, an Alk inhibitor, a phosphatidylinositol 3-kinase inhibitor, an Akt inhibitor, an mTOR inhibitor, a VEGF inhibitor, a CD73 inhibitor, a PARP inhibitor, a PD-1 inhibitor, a diphosphatidylglycol 3-kinase/mTOR inhibitor, and a combination thereof.

In one or more embodiments of the present invention, the antibody-drug conjugate comprises a drug selected from the group consisting of: maytansine, monomethyl auristatin E, calicheamicin, esperamicin, and a radioisotope chelating agent.

The CD73 inhibitor includes, but is not limited to, one or more of BMS-986179, MEDI9447, NZV930, CPI-006, AB680, LY-3475070, TJ004309[3], ORIC-533, IPH5301AB680 and LY-3475070.

The PARP inhibitor is selected from one or more of etoposide, olaparib, rucaparib, niraparib, talazoparib, fluzoparib, veliparib ER, ABT-472, ABT-767, Stenoparib, AST-6828, AG-PD, ANG-2864, ANG-3038, ANG-3186, AZD-5305, AZ-0108, AZD-2461, AMXI-5001, AMXI-2001, AMXI-3001, AMXI-7001, AMXI-9001, pamiparib, ZYTP-1, CK-102, XZ-120312, YHP-743, iobenguane I 131, rucaparib camsylate, CVL-218, CPH-101, CPH-102, CBX-11, CBX-15, minocycline, DB-207, DPS-102, E-7016, iobenguane I 131, MK-2512, HCX-014, HWH-340, IDX-1197, IDX-1197, senaparib, IMP-04100, IMP-04111, IMP-04149, IMP-04249, IMP-04307, IMP-04356, JPI-289, JPI-547, JPI-283, fluzoparib, GT-1620, iobenguane I 131, DR-2313, MP-124, H-10, NT-125, BGP-15, NMSP-293, NMSP-293, NMSP-118, NMSP-648, NMSP-914, DB-207, NUV-1156, NUV-1176, JPI-289, Stenoparib, OX-401, NU-1025, NU-1085, PLX-376, R-554, RBN-2397, RBN-012759, PJ-34, INO-1001, WW-46, BSI-401, iniparib, SOMCL-9112, SC-10914, HTMC-0435, SRX-3128, TSL-1502, PJ-34, CEP-8983, CK-102, THG-009, talazoparib SR, L-2286, mitoparib and WB-1340.

In one or more embodiments of the present invention, the tumor is selected from one or more of the following:
cervical cancer (e.g., metastatic cervical cancer), endometrial cancer, lung cancer such as small cell lung cancer and non-small cell lung cancer (e.g., squamous non-small cell lung cancer or non-squamous non-small cell lung cancer), throat cancer, esophageal cancer, esophageal squamous cancer, thyroid cancer, mesothelioma, gastrointestinal cancer such as gastric cancer ((including microsatellite stability (MSS) and mismatch repair dysfunction/microsatellite high instability (dMMR/MSI-H) type), e.g., advanced gastric cancer, gastric adenocarcinoma or gastroesophageal junction adenocarcinoma), and intestinal cancer (e.g., rectal cancer, colon cancer, colorectal cancer (including microsatellite stability (MSS) and mismatch repair dysfunction/microsatellite high instability (dMMR/MSI-H) type)), liver cancer (e.g., hepatocellular carcinoma, hepatobiliary cancer), biliary tract cancer (e.g., cholangiocarcinoma and gallbladder cancer), pancreatic cancer, pancreas cancer, renal cancer, ovarian cancer (e.g., advanced ovarian cancer), fallopian tube cancer, anal epidermoid carcinoma, peritoneal cancer, glioma, neuroglioma, recurrent glioma, skin cancer, melanoma, hematological malignancy (such as leukemia (e.g., acute myeloid leukemia)), lymphoma (e.g., Hodgkin's lymphoma, non-Hodgkin's lymphoma), multiple myeloma, B-lymphoma (e.g., plasma cell carcinoma), bone cancer, sarcoma (e.g., leiomyosarcoma, rhabdomyosarcoma), osteosarcoma, chondrosarcoma, neuroblastoma, myeloma (e.g., multiple myeloma), large cell neuroendocrine cancer, urothelial carcinoma (e.g., upper urothelial carcinoma or bladder cancer), prostate cancer (including metastatic castration-resistant prostate cancer (mCRPC)), testicular cancer, triple-negative breast cancer, peripheral T-cell lymphoma, nasopharyngeal cancer, microsatellite high instability (MSI-H) or mismatch repair dysfunction (dMMR) type solid tumor, head and neck cancer, brain cancer (e.g., aggressive brain cancer), squamous cell carcinoma, basal cell carcinoma, adenoma (e.g., breast cancer, thymus cancer, ileocecal adenocarcinoma, ampullate adenocarcinoma, pancreatic ductal adenocarcinoma, mucinous or serous cystadenocarcinoma), chorionic epithelioma, malignant hydatidiform mole, malignant sertoli cell-stromal cell tumor, malignant granulocytoma, dysgerminoma, glioblastoma, mycosis, Merkel cell carcinoma, intrahepatic bile duct carcinoma, Merkel cell carcinoma, squamous cell anorectal cancer, squamous cell carcinoma of the tongue, squamous cell carcinoma of the head and neck, and other hematological malignant tumors.

In one or more embodiments of the present invention, the anti-PD-1-anti-VEGFA bispecific antibody comprises:
a first protein functional region targeting PD-1, and
a second protein functional region targeting VEGF A;
wherein the first protein functional region is a single chain antibody, and the second protein functional region is an immunoglobulin; or the first protein functional region is an immunoglobulin, and the second protein functional region is a single chain antibody,
wherein,
a heavy chain variable region of the immunoglobulin comprises: HCDR1-HCDR3 contained in a heavy chain variable region having amino acid sequences set forth in SEQ ID NO: 27 (preferably HCDR1-HCDR3 set forth in SEQ ID NOs: 31-33, respectively, according to the IMGT numbering system), and a light chain variable region thereof comprises: LCDR1-LCDR3 contained in a light chain variable region having amino acid sequences set forth in SEQ ID NO: 29 (preferably LCDR1-LCDR3 set forth in SEQ ID NOs: 34-36, respectively, according to the IMGT numbering system);
a heavy chain variable region of the single chain antibody comprises: HCDR1-HCDR3 contained in a heavy chain variable region having amino acid sequences set forth in SEQ ID NO: 37 (preferably HCDR1-HCDR3 set forth in SEQ ID NOs: 41-43, respectively, according to the IMGT numbering system), and a light chain variable region thereof comprises: LCDR1-LCDR3 contained in a light chain variable region having amino acid sequences set forth in SEQ ID NO: 39 (preferably LCDR1-LCDR3 set forth in SEQ ID NOs: 44-46, respectively, according to the IMGT numbering system);
   or
a heavy chain variable region of the immunoglobulin comprises: HCDR1-HCDR3 contained in a heavy chain variable region having amino acid sequences set forth in SEQ ID NO: 37 (preferably HCDR1-HCDR3 set forth in SEQ ID NOs: 41-43, respectively, according to the IMGT numbering system), and a light chain variable region thereof comprises: LCDR1-LCDR3 contained in a light chain variable region having amino acid sequences set forth in SEQ ID NO: 39 (preferably LCDR1-LCDR3 set forth in SEQ ID NOs: 44-46, respectively, according to the IMGT numbering system);
a heavy chain variable region of the single chain antibody comprises: HCDR1-HCDR3 contained in a heavy chain variable region having amino acid sequences set forth in SEQ ID NO: 27 (preferably HCDR1-HCDR3 set forth in SEQ ID NOs: 31-33, respectively, according to the IMGT numbering system), and a light chain variable region thereof comprises: LCDR1-LCDR3 contained in a light chain variable region having amino acid sequences set forth in SEQ ID NO: 29 (preferably LCDR1-LCDR3 set forth in SEQ ID NOs: 34-36, respectively, according to the IMGT numbering system); and
preferably, the immunoglobulin is of human IgG1 subtype.

In one or more embodiments of the present invention, for the bispecific antibody, according to the EU numbering system, the heavy chain constant region of the immunoglobulin has the following mutations:
L234A and L235A; or
L234A and G237A; or
L235A and G237A;
   or
L234A, L235A and G237A.

In the present invention, letters before the position number represent amino acids before mutation, and letters after the position number represent amino acids after mutation, unless otherwise specified.

In one or more embodiments of the present invention, for the bispecific antibody, according to the EU numbering system, the heavy chain constant region of the immunoglobulin has or further has one or more mutations selected from:
N297A, D265A, D270A, P238D, L328E, E233D, H268D, P271G, A330R, C226S, C229S, E233P, P331S, S267E, L328F, A330L, M252Y, S254T, T256E, N297Q, P238S, P238A, A327Q, A327G, P329A, K322A, T394D, G236R, G236A, L328R, A330S, P331S, H268A, E318A and K320A.

In one or more embodiments of the present invention, for the bispecific antibody,
an amino acid sequence of the heavy chain variable region of the immunoglobulin is set forth in SEQ ID NO: 27, and an amino acid sequence of the light chain variable region of the immunoglobulin is set forth in SEQ ID NO: 29; an amino acid sequence of the heavy chain variable region of the single chain antibody is set forth in SEQ ID NO: 37, and an amino acid sequence of the light chain variable region of the single chain antibody is set forth in SEQ ID NO: 39.

In one or more embodiments of the present invention, the bispecific antibody has a heavy chain amino acid sequence set forth in SEQ ID NO: 23, and a light chain amino acid sequence set forth in SEQ ID NO: 25. Preferably, a heavy chain of the bispecific antibody is encoded by a nucleotide sequence set forth in SEQ ID NO: 24, and a light chain thereof is encoded by an amino acid sequence set forth in SEQ ID NO: 26.

In some embodiments of the present invention, for the bispecific antibody, the single chain antibody is linked to the C terminus of the heavy chain of the immunoglobulin. Since an immunoglobulin has two heavy chains, two single chain antibody molecules are linked to one immunoglobulin molecule. Preferably, the two single chain antibody molecules are identical.

In some embodiments of the present invention, for the bispecific antibody, two single chain antibodies are present, and one terminus of each single chain antibody is linked to the C terminus or the N terminus of one of the two heavy chains of the immunoglobulin.

In some embodiments of the present invention, a disulfide bond is present between the VH and VL of the single chain antibody. Methods for introducing a disulfide bond between the VH and VL of an antibody are well known in the art, see, for example, US 5,747,654; Rajagopal et al., Prot. Engin. 10(1997)1453-1459; Reiter et al., Nat. Biotechnol. 14(1996)1239-1245; Reiter et al., Protein Engineering 8(1995)1323-1331; Webber et al., Molecular Immunology 32(1995)249-258; Reiter et al., Immunity 2(1995)281-287; Reiter et al., JBC 269(1994)18327-18331; Reiter et al., Inter J. of Cancer 58(1994)142-149; or Reiter et al., Cancer Res. 54(1994)2714-2718, which are incorporated herein by reference.

In one or more embodiments of the present invention, for the bispecific antibody, the first protein functional region is linked to the second protein functional region either directly or via a linker fragment; and/or the heavy chain variable region of the single chain antibody is linked to the light chain variable region of the single chain antibody either directly or via a linker fragment.

In one or more embodiments of the present invention, for the bispecific antibody, the linker fragment is (GGGGS)n, wherein n is a positive integer, preferably, n is 1, 2, 3, 4, 5 or 6.

In one or more embodiments of the present invention, in the bispecific antibody, the numbers of the first protein functional region and the second protein functional region are each independently 1, 2 or more.

In one or more embodiments of the present invention, for the bispecific antibody, the single chain antibody is linked to the C terminus of the heavy chain of the immunoglobulin.

In one or more embodiments of the present invention, the first protein functional region is linked to the second protein functional region via a first linker fragment; and the heavy chain variable region of the single chain antibody is linked to the light chain variable region of the single chain antibody via a second linker fragment; the first linker fragment and the second linker fragment are the same or different;
preferably, amino acid sequences of the first linker fragment and second linker fragment are independently selected from SEQ ID NO: 47 and SEQ ID NO: 48;
preferably, amino acid sequences of the first linker fragment and second linker fragment are set forth in SEQ ID NO: 48.

In one or more embodiments of the present invention, the bispecific antibody is a monoclonal antibody.

In one or more embodiments of the present invention, the bispecific antibody is a humanized antibody.

Another aspect of the present invention relates to a unit formulation, preferably used for treating a tumor, and comprising 1-10000 mg (preferably 10-1000 mg, preferably 50-500 mg, 100-400 mg, 150-300 mg, 150-250 mg or 200 mg) of the anti-CD73 antibody according to any one of the aspects of the present invention and 1-10000 mg (preferably 1-1000 mg, preferably 50-500 mg, 100-400 mg, 150-300 mg, 150-250 mg, 200 mg or 100 mg) of the anti-PD-1-anti-VEGFA bispecific antibody according to any one of the aspects of the present invention, and optionally one or more of the chemotherapeutic drugs (such as a platinum-based drug and/or a fluorouracil antineoplastic drug) according to the present invention; wherein the anti-CD73 antibody, the anti-PD-1-anti-VEGFA bispecific antibody and the chemotherapeutic drug are packaged separately.

The present invention relates to a method for preventing or treating cancer or a tumor, wherein the method comprises administering to a subject in need thereof one or more unit formulations according to the present invention, preferably the anti-PD-1-anti-VEGFA bispecific antibody, the anti-CD73 antibody and the chemotherapeutic drug in the unit formulation are each administered separately.

Another aspect the present invention relates to a single dose unit, preferably used for treating a tumor, and comprising 0.1-10000 mg (preferably 1-1000 mg, preferably 50-500 mg, 100-400 mg, 150-300 mg, 150-250 mg, 200 mg or 100 mg) of the anti-CD73 antibody according to any one of the aspects of the present invention and 0.1-10000 mg (preferably 1-1000 mg, preferbly 50-500 mg, 100-400 mg, 150-300 mg, 150-250 mg, 200 mg or 100 mg) of the anti-PD-1-anti-VEGFA bispecific antibody according to any one of the aspects of the present invention.

In one or more embodiments of the present invention, the anti-CD73 antibody, the anti-PD-1-anti-VEGFA bispecific antibody and/or the chemotherapeutic drug is in a form suitable for intravenous injection or intravenous drip infusion, preferably in a liquid form.

In one or more embodiments of the present invention, the step in which an effective amount of the anti-CD73 antibody according to any one of the aspects of the present invention and/or the anti-PD-1-anti-VEGFA bispecific antibody according to any one of the aspects of the present invention is administered to a subject is before or after a surgical treatment and/or before or after a radiotherapy.

In one or more embodiments of the present invention, the unit dose of the anti-CD73 antibody according to any one of the aspects of the present invention and/or the anti-PD-1-anti-VEGFA bispecific antibody according to any one of the aspects of the present invention is 0.1-100 mg, preferably 1-10 mg per kg body weight; alternatively, the unit dose of the anti-CD73 antibody according to any one of the aspects of the present invention and/or the anti-PD-1-anti-VEGFA bispecific antibody according to any one of the aspects of the present invention is 10-1000 mg, preferably 50-500 mg, 100-400 mg, 150-300 mg, 150-250 mg or 200 mg in each subject,
preferably, the dose is given from twice daily to about once every other day, or once every 3 days, 4 days, 5 days, 6 days, 10 days, 1 week, 2 weeks, 3 weeks, 4 weeks, 5 weeks or 6 weeks;
preferably, the route of administration is intravenous drip infusion or intravenous injection.

In the present invention, unless otherwise defined, the scientific and technical terms used herein have the meanings generally understood by those skilled in the art. In addition, the laboratory operations of cell culture, molecular genetics, nucleic acid chemistry and immunology used herein are the routine procedures widely used in the corresponding fields. Meanwhile, in order to better understand the present invention, the definitions and explanations of the relevant terms are provided below.

As used herein, the term EC₅₀ refers to the concentration for 50% of maximal effect, i.e., the concentration that can cause 50% of the maximal effect.

As used herein, the term "antibody" refers to an immunoglobulin molecule that generally consists of two pairs of polypeptide chains (each pair with one "light" (L) chain and one "heavy" (H) chain). Antibody light chains are classified as κ and λ light chains. Heavy chains are classified as µ, δ, γ, α, or ε. Isotypes of antibodies are defined as IgM, IgD, IgG, IgA, and IgE. In light chains and heavy chains, the variable region and constant region are linked by a "J" region of about 12 or more amino acids, and the heavy chain further comprises a "D" region of about 3 or more amino acids. Each heavy chain consists of a heavy chain variable region (V_{H}) and a heavy chain constant region (C_{H}). The heavy chain constant region consists of 3 domains (C_{H1}, C_{H2}, and C_{H3}). Each light chain consists of a light chain variable region (V_{L}) and a light chain constant region (C_{L}). The light chain constant region consists of one domain C_{L}. The constant region of the antibody can mediate the binding of immunoglobulins to host tissues or factors, including the binding of various cells of the immune system (e.g., effector cells) to the first component (C1q) of classical complement system. The V_{H} and V_{L} regions can be further subdivided into hypervariable regions (called complementarity determining regions, or CDRs) and conservative regions called framework regions (FRs) that are distributed between the CDRs. Each V_{H} and V_{L} consists of 3 CDRs and 4 FRs arranged from the amino terminus to the carboxyl terminus in the following order: FR1, CDR1, FR2, CDR2, FR3, CDR3, FR4. The variable regions (V_{H} and V_{L}) of each heavy chain/light chain pair form antigen-binding sites, respectively. The assignment of amino acids to the regions or domains is based on Kabat Sequences of Proteins of Immunological Interest (National Institutes of Health, Bethesda, M.d. (1987 and 1991)), or Chothia & Lesk, J. Mol. Biol., 1987; 196:901-917; Chothia et al., Nature, 1989; 342:878-883 or the definition of IMGT numbering system, see Ehrenmann, Francois, Quentin Kaas, and Marie-Paule Lefranc., "IMGT/3Dstructure-DB and IMGT/DomainGapAlign: a database and a tool for immunoglobulins or antibodies, T cell receptors, MHC, IgSF and MhcSF.", Nucleic acids research, 2009; 38(suppl_1):D301-D307. The term "antibody" is not limited by any specific method for producing the antibody. For example, the antibody includes, in particular, a recombinant antibody, a monoclonal antibody and a polyclonal antibody. The antibody can be antibodies of different isotypes, such as IgG (e.g., subtypes IgG1, IgG2, IgG3 or IgG4), IgA1, IgA2, IgD, IgE or IgM.

Given the known sequences of the heavy and light chain variable regions of an antibody, there are several methods for determining the CDRs of the antibody, including the Kabat, IMGT, Chothia, and AbM numbering systems. However, the application of all the definitions of CDRs for an antibody or its variant shall fall within the scope of the terms defined and used herein. If an amino acid sequence of the variable region of the antibody is known, those skilled in the art can generally determine a particular CDR, without relying on any experimental data beyond the sequence itself.

As used herein, the terms "mAb" and "monoclonal antibody" refer to an antibody or a fragment of an antibody that is derived from a group of highly homologous antibodies, i.e., from a group of identical antibody molecules, except for natural mutations that may occur spontaneously. The monoclonal antibody is highly specific for a single epitope on an antigen. The polyclonal antibody, relative to the monoclonal antibody, generally comprises at least 2 or more different antibodies which generally recognize different epitopes on an antigen. Monoclonal antibodies can generally be obtained using hybridoma technology first reported by Kohler et al. (Köhler G, Milstein C., Continuous cultures of fused cells secreting antibody of predefined specificity [J]. Nature, 1975; 256(5517):495), but can also be obtained using recombinant DNA technology (see, e.g., U.S. Patent 4,816,567).

As used herein, the term "humanized antibody" refers to an antibody or antibody fragment obtained when all or a part of the CDRs of a human immunoglobulin (receptor antibody) is replaced by the CDRs of a non-human antibody (donor antibody), wherein the donor antibody may be a non-human (e.g., mouse, rat or rabbit) antibody having expected specificity, affinity or reactivity. In addition, some amino acid residues in the framework regions (FRs) of the receptor antibody can also be replaced by the amino acid residues of corresponding non-human antibodies or by the amino acid residues of other antibodies to further improve or optimize the performance of the antibody. For more details on humanized antibodies, see, e.g., Jones et al., Nature, 1986; 321:522-525; Reichmann et al., Nature, 1988; 332:323-329; Presta, Curr. Op. Struct. Biol., 1992; 2:593-596; and Clark M., Antibody humanization: a case of the "Emperor's new clothes" [J]. Immunol. Today, 2000; 21(8):397-402.

As used herein, the term "isolated" refers to obtaining by artificial means from a natural state. If a certain "isolated" substance or component is present in nature, it may be the case that a change occurs in its natural environment, or that it is isolated from the natural environment, or both. For example, a certain non-isolated polynucleotide or polypeptide naturally occurs in a certain living animal, and the same polynucleotide or polypeptide with high purity isolated from such a natural state is referred to as an isolated polynucleotide or polypeptide. The term "isolated" does not exclude the existence of artificial or synthetic substances or other impurities that do not affect the activity of the substance.

As used herein, the term "vector" refers to a nucleic acid vehicle into which a polynucleotide can be inserted. When a vector allows the expression of the protein encoded by the inserted polynucleotide, the vector is referred to as an expression vector. The vector can be introduced into a host cell by transformation, transduction or transfection, such that the genetic substance elements carried by the vector can be expressed in the host cell. Vectors are well known to those skilled in the art, including but not limited to: plasmids; phagemids; cosmids; artificial chromosomes, such as yeast artificial chromosome (YAC), bacterial artificial chromosome (BAC), or P1-derived artificial chromosome (PAC); phages, such as lambda phages or M13 phages; and animal viruses. Animal viruses that can be used as vectors include, but are not limited to retroviruses (including lentiviruses), adenoviruses, adeno-associated viruses, herpes viruses (such as herpes simplex virus), poxviruses, baculoviruses, papillomaviruses, and papovaviruses (such as SV40). A vector may comprise a variety of elements that control expression, including, but not limited to, promoter sequences, transcription initiation sequences, enhancer sequences, selection elements and reporter genes. In addition, the vector may further comprise a replication initiation site.

As used herein, the term "host cell" refers to cells to which vectors can be introduced, including, but not limited to, prokaryotic cells such as *E. coli* or *bacillus subtilis,* fungal cells such as yeast cells or *aspergillus,* insect cells such as S2 drosophila cells or Sf9, or animal cells such as fibroblasts, CHO cells, COS cells, NSO cells, HeLa cells, GS cells, BHK cells, HEK 293 cells, or human cells.

As used herein, the term "specific binding" refers to a non-random binding reaction between two molecules, such as a reaction between an antibody and an antigen it targets. In some embodiments, an antibody specifically binding to an antigen (or an antibody specific to an antigen) means that the antibody binds to the antigen with an affinity (K_{D}) of less than about 10⁻⁵ M, e.g., less than about 10⁻⁶ M, 10⁻⁷ M, 10⁻⁸ M, 10⁻⁹ M or 10⁻¹⁰ M or less.

As used herein, the term "K_{D}" refers to a dissociation equilibrium constant for a specific antibody-antigen interaction, which is used to describe the binding affinity between the antibody and the antigen. A smaller dissociation equilibrium constant indicates a stronger antibody-antigen binding and a higher affinity between the antibody and the antigen. Generally, antibodies bind to antigens (e.g., PD-1 protein) with a dissociation equilibrium constant (K_{D}) of less than about 10⁻⁵ M, e.g., less than about 10⁻⁶ M, 10⁻⁷ M, 10⁻⁸ M, 10⁻⁹ M or 10⁻¹⁰ M or less. K_{D} can be determined using methods known to those skilled in the art, e.g., using a Fortebio molecular interaction instrument.

As used herein, the terms "monoclonal antibody" and "mAb" have the same meaning and can be used interchangeably; the terms "polyclonal antibody" and "pAb" have the same meaning and can be used interchangeably; the terms "polypeptide" and "protein" have the same meaning and can be used interchangeably. Besides, as used herein, amino acids are generally represented by single-letter and three-letter abbreviations known in the art. For example, alanine can be represented by A or Ala.

As used herein, the term "pharmaceutically acceptable carrier and/or excipient" refers to a carrier and/or excipient that is pharmacologically and/or physiologically compatible with the subject and the active ingredient. Such carriers and/or excipients are well known in the art (see, e.g., *Remington's Pharmaceutical Sciences,* edited by Gennaro AR, 19^{th} Ed., Pennsylvania: Mack Publishing Company, 1995), including but not limited to: pH regulators, surfactants, adjuvants and ionic strength enhancers. For example, the pH regulators include, but are not limited to, phosphate buffer; the surfactants include, but are not limited to, cationic, anionic or non-ionic surfactants, such as Tween-80; the ionic strength enhancers include, but are not limited to, sodium chloride.

As used herein, the term "effective amount" refers to an amount sufficient to obtain or at least partially obtain a desired effect. For example, a prophylactically effective amount against a disease (e.g., a tumor) refers to an amount sufficient to prevent, stop, or delay the onset of the disease (e.g., a tumor); a therapeutically effective amount refers to an amount sufficient to cure or at least partially stop a disease and complications thereof in patients suffering from the disease.

The term "single dose unit" means a single pharmaceutical dosage form, such as an injection, e.g. placed in an ampoule, comprising the anti-CD73 antibody and the anti-PD-1-anti-VEGFA bispecific antibody according to the present invention to be administered to a subject at time points of a regimen, preferably per kg body weight of the subject. In a specific embodiment of the present invention, the regimen comprises, for example, administration of a single dose unit according to an administration cycle of from twice daily to about once every other day, or once every 3 days, 4 days, 5 days, 6 days, 10 days, 1 week, 2 weeks, 3 weeks, 4 weeks, 5 weeks or 6 weeks.

In the present invention, the terms "first" (e.g., first protein functional region or first linker fragment) and "second" (e.g., second protein functional region or second linker fragment) are used for distinguishing or clarity in expression and do not carry typical sequential meanings, unless otherwise specified.

A "therapeutically effective amount" or "therapeutically effective dose" of a drug or therapeutic agent is any amount of a drug that, when used alone or in combination with another therapeutic agent, protects a subject from the onset of a disease or promotes disease regression as evidenced by reduction in the severity of disease symptoms, increase in the frequency and duration of disease symptom-free periods, or the prevention of damage or disability caused by the affliction of the disease. The ability of a therapeutic to promote disease regression can be evaluated using a variety of methods known to those skilled in the art, such as in a human subject in clinical trials, in an animal model system that predicts the efficacy in humans, or by determining the activity of the drug in an *in vitro* assay.

The "prophylactically effective amount" of a drug refers to any amount of a drug that inhibits the occurrence or recurrence of cancer when administered, alone or in combination with an antineoplastic, to a subject at risk of developing a cancer (e.g., a subject having a premalignant condition) or a subject at risk of recurrence of a cancer. In some embodiments, the prophylactically effective amount completely prevents the occurrence or recurrence of a cancer. "Inhibiting" the occurrence or recurrence of cancer means reducing the possibility of the occurrence or recurrence of a cancer or completely preventing the occurrence or recurrence of a cancer.

### Beneficial effects:

the monoclonal antibody of the present invention can specifically bind to CD73 well, and can effectively inhibit the enzyme activity reaction of CD73 in a non-substrate competition mode, reduce the production of adenosine, and promote the activity of T cells and the tumor inhibitory effect. Meanwhile, the antibody in combination with the anti-PD-1-anti-VEGFA bispecific antibody of the present invention has a pharmacological effect of effectively inhibiting tumor growth, which is superior to that of either the anti-VEGFA/PD-1 bispecific antibody or the anti-CD73 antibody alone.

### BRIEF DESCRIPTION OF THE DRAWINGS

FIG. 1 shows the fitting curve of dynamic affinity data of 19F3H2L3(hG1DM) binding to C1q.
FIG. 2 shows the fitting curve of dynamic affinity data of MEDI9447 binding to C1q.
FIG. 3 shows the fitting curve of dynamic affinity data of wild-type IgG1 antibody binding to C1q.
FIG. 4 shows the fitting curve of dynamic affinity data of 19F3H2L3(hG1DM) binding to FcyRIIIa.
FIG. 5 shows the fitting curve of dynamic affinity data of MEDI9447 binding to FcyRIIIa.
FIG. 6 shows the fitting curve of dynamic affinity data of wild-type IgG1 antibody binding to FcyRIIIa.
FIG. 7 shows the anti-CD73 antibody effectively inhibiting CD73 enzymatic activity.
FIG. 8 shows the effect of 19F3H2L3(hG1DM) antibody in combination with the anti-PD-1-anti-VEGFA bispecific antibody VP101(hG1DM) on B6-hPD1/hPDL1/hCD73 mouse MC38-hPDL1/hCD73 tumor model.
FIG. 9 shows the changes in the body weight of the B6-hPD1/hPDL1/hCD73 mouse MC38-hPDL1/hCD73 tumor model by 19F3H2L3(hG1DM) antibody in combination with the anti-PD-1-anti-VEGFA bispecific antibody VP101(hG1DM).
FIG. 10 shows that the anti-CD73 specific antibody in combination with anti-PD-1-anti-VEGFA bispecific antibody promotes secretion of the cytokine IFN-γ.

### DETAILED DESCRIPTION

The embodiments of the present invention will be described in detail below with reference to the examples. Those skilled in the art will appreciate that the following examples are only for illustrating the present invention, and should not be construed as limitations to the scope of the present invention. Examples where the specific technologies or conditions are not specified are performed according to the technologies or conditions described in the publications of the art (e.g., see, Guide to Molecular Cloning Experiments, authored by J. Sambrook et al., and translated by Huang Peitang et al., third edition, Science Press) or according to the product instruction. Reagents or instruments used are commercially available conventional products if the manufacturers thereof are not specified.

In the following examples of the present invention, BALB/c mice used were purchased from Guangdong Medical Experimental Animal Center.

In the following examples of the present invention, the positive control antibody MEDI9447 (Oleclumab) used was produced by Akeso Biopharma, Inc., the sequence of which was identical to the antibody sequence described in International Nonproprietary Names for Pharmaceutical Substances (INN) publicly published by Melmmune Limited at the WHO site (World Health Organization (2016). "International Nonproprietary Names for Pharmaceutical Substances (INN). Proposed INN: List 116" (PDF). WHO Drug Information. 30(4), P661-662).

In the following examples of the present invention, the combination anti-PD-1-anti-VEGFA bispecific antibody VP101(hG1DM) used was produced by Akeso Biopharma, Inc., the sequence and preparation of which is referred to antibody VP101(hG1DM) described in Publication Patent CN112830972A, wherein an amino acid full-length sequence of a heavy chain of VP101(hG1DM) is set forth in SEQ ID NO: 23 and an amino acid full-length sequence of a light chain thereof is set forth in SEQ ID NO: 25. VP101(hG1DM) has a structure of IgG-scFv, in which the IgG part is an anti-VEGFA antibody and the scFv part is an anti-PD-1 antibody, wherein
the anti-VEGFA antibody has an HCDR1 sequence set forth in SEQ ID NO: 31, an HCDR2 sequence set forth in SEQ ID NO: 32, an HCDR3 sequence set forth in SEQ ID NO: 33, and a VH sequence set forth in SEQ ID NO: 27, and the anti-VEGFA antibody has an LCDR1 sequence set forth in SEQ ID NO: 34, an LCDR2 sequence set forth in SEQ ID NO: 35, an LCDR3 sequence set forth in SEQ ID NO: 36, and a VL sequence set forth in SEQ ID NO: 29; and
the anti-PD1 antibody has an HCDR1 sequence set forth in SEQ ID NO: 41, an HCDR2 sequence set forth in SEQ ID NO: 42, an HCDR3 sequence set forth in SEQ ID NO: 43, and a VH sequence set forth in SEQ ID NO: 37, and
the anti-PD1 antibody has an LCDR1 sequence set forth in SEQ ID NO: 44, an LCDR2 sequence set forth in SEQ ID NO: 45, an LCDR3 sequence set forth in SEQ ID NO: 46, and a VL sequence set forth in SEQ ID NO: 39.

In the following examples of the present invention, the heavy chain constant region of the positive control antibody wild IgG1 control antibody used was Ig gamma-1 chain C region, ACCESSION: P01857; the light chain constant region was Ig kappa chain C region, ACCESSION: P01834.

In the following examples of the present invention, C1q used was purchased from fizgerald, Cat No. A16050201;
in the following examples of the present invention, FcyRIIIa-bio used was purchased from Sino Biological, Cat No. LC09JA0407;
in the following examples of the present invention, the CD73 (5'-nuclease) specific inhibitor APCP (alpha, beta-methylene adenosine-5'-diphosphate, 5'-α, β-methylene-adenosine diphosphate) used was derived from Sigma, Cat No. M3763-10MG.

In the following examples of the present invention, the sequence of the isotype control antibody, human anti-hen egg lysozyme IgG (i.e., anti-HEL antibody, or human IgG, abbreviated as hIgG, or isotype control) was derived from the variable region sequence of the Fab F10.6.6 sequence in the study reported by Acierno et al., entitled "Affinity maturation increases the stability and plasticity of the Fv domain of anti-protein antibodies" (Acierno et al., J Mol Biol., 2007; 374(1):130-146).

### Example 1: Preparation of Anti-CD73 Antibody 19F3

### 1. Preparation of hybridoma cell line LT014

The antigen used to prepare the anti-CD73 antibody was human NT5E-His (for NT5E, GenbankID: NP _002517.1, position: 1-552, prepared by Akeso Biopharma, Inc.). Spleen cells of immunized mice were taken to fuse with myeloma cells of the mice to prepare hybridoma cells. Hybridoma cells were screened by indirect ELISA using human NT5E-Biotin (for NT5E, GenbankID: NP_002517.1, position: 1-552, prepared by Akeso Biopharma, Inc.) as an antigen, and hybridoma cells capable of secreting an antibody specifically binding to CD73 were obtained. The hybridoma cells obtained by screening were subjected to limiting dilution to obtain a stable hybridoma cell line. The hybridoma cell line was named as hybridoma cell line LT014, and the monoclonal antibody secreted therefrom was named as 19F3.

The hybridoma cell line LT014 (also called CD73-19F3) was deposited at China Center for Type Culture Collection (CCTCC) on June 21, 2018 with the accession number CCTCC NO: C2018137, the collection address being Wuhan University, Wuhan, China, postal code: 430072.

### 2. Preparation of anti-CD73 antibody 19F3

The LT014 cell line prepared above was cultured with a chemical defined medium (CD medium, containing 1% Penicillin-Streptomycin) in a 5% COz cell incubator at 37 °C. After 7 days, the cell culture supernatant was collected, subjected to high-speed centrifugation and vacuum filtration through a microfiltration membrane, and purified by using a HiTrap protein A HP column to obtain an antibody 19F3.

### Example 2: Sequence Analysis of Anti-CD73 Antibody 19F3

mRNA was extracted from the cell line LT014 cultured in Example 1 according to the method described in the manual of RNAprep pure Cell/Bacteria Kit (Tiangen, Cat. No. DP430).

cDNA was synthesized according to the manual of Invitrogen SuperScript^{®} III First-Strand Synthesis System for RT-PCR and amplified by PCR.

The PCR-amplified products were directly subjected to TA cloning according to the manual of the pEASY-T1 Cloning Kit (Transgen CT101).

The TA-cloned products were directly sequenced, and the sequencing results of the anti-CD73 antibody 19F3 were as follows:
The nucleic acid sequence of the heavy chain variable region is set forth in SEQ ID NO: 1 with a length of 363 bp.
The encoded amino acid sequence is set forth in SEQ ID NO: 2 with a length of 121 aa;
wherein the sequences of heavy chains CDR1, CDR2 and CDR3 are set forth in SEQ ID NOs: 15, 16 and 17, respectively.
The nucleic acid sequence of the light chain variable region is set forth in SEQ ID NO: 3 with a length of 339 bp.
The encoded amino acid sequence is set forth in SEQ ID NO: 4 with a length of 113 aa;
wherein the sequences of light chains CDR1, CDR2 and CDR3 are set forth in SEQ ID NOs: 18, 19 and 20, respectively.

### Example 3. Design and Preparation of Light and Heavy Chains of Humanized Anti-Human CD73 Antibodies

The variable region sequences of 19F3H1L 1 (hG 1DM), 19F3H2L2(hG1DM) and 19F3H2L3(hG1DM) were obtained by computer modeling antibody models according to model design mutations based on the sequences of the antibody 19F3 obtained in Example 2 and based on the three-dimensional crystal structure of human CD73 protein (Hage T, Reinemer P, Sebald W., Crystals of a 1:1 complex between human interleukin-4 and the extracellular domain of its receptor alpha chain. Eur J Biochem. 1998; 258(2):831-6);
the designed variable region sequences of the humanized antibody were as follows:

### (1) Heavy and light chain variable region sequences of humanized monoclonal antibody 19F3H1L1(hG1DM)

The nucleic acid sequence of the heavy chain variable region is set forth in SEQ ID NO: 5 with a length of 363 bp.

The coded amino acid sequence is set forth in SEQ ID NO: 6 with a length of 121 aa, while the sequences of heavy chains CDR1, CDR2 and CDR3 are set forth in SEQ ID NOs: 15, 16 and 17, respectively.

The nucleic acid sequence of the light chain variable region is set forth in SEQ ID NO: 7 with a length of 339 bp.

The coded amino acid sequence is set forth in SEQ ID NO: 8 with a length of 113 aa, while the sequences of light chains CDR1, CDR2 and CDR3 are set forth in SEQ ID NOs: 18, 19 and 20, respectively.

### (2) Heavy and light chain variable region sequences of humanized monoclonal antibody 19F3H2L2(hG1DM)

The nucleic acid sequence of the heavy chain variable region is set forth in SEQ ID NO: 9 with a length of 363 bp.

The coded amino acid sequence is set forth in SEQ ID NO: 10 with a length of 121 aa, while the sequences of heavy chains CDR1, CDR2 and CDR3 are set forth in SEQ ID NOs: 15, 16 and 17, respectively.

The nucleic acid sequence of the light chain variable region is set forth in SEQ ID NO: 11 with a length of 339 bp.

The coded amino acid sequence is set forth in SEQ ID NO: 12 with a length of 113 aa, while the sequences of light chains CDR1, CDR2 and CDR3 are set forth in SEQ ID NOs: 18, 19 and 20, respectively.

### (3) Heavy and light chain variable region sequences of humanized monoclonal antibody 19F3H2L3(hG1DM)

The nucleic acid sequence of the heavy chain variable region is set forth in SEQ ID NO: 9 with a length of 363 bp.

The coded amino acid sequence is set forth in SEQ ID NO: 10 with a length of 121 aa, while the sequences of heavy chains CDR1, CDR2 and CDR3 are set forth in SEQ ID NOs: 15, 16 and 17, respectively.

The nucleic acid sequence of the light chain variable region is set forth in SEQ ID NO: 13 with a length of 339 bp.

The coded amino acid sequence is set forth in SEQ ID NO: 14 with a length of 113 aa, while the sequences of light chains CDR1, CDR2 and CDR3 are set forth in SEQ ID NOs: 18, 19 and 20, respectively.

### 3. Preparation of humanized 19F3H1L 1 (hG 1DM), 19F3H2L2(hG1DM) and 19F3H2L3 (hG1DM)

The light chain constant regions of 19F3H1L1(hG1DM), 19F3H2L2(hG1DM) and 19F3H2L3(hG1DM) are the Ig kappa chain C region, ACCESSION: P01834.

On the basis of Ig gamma-1 chain C region, ACCESSION: P01857, humanized antibodies were obtained by introducing a leucine-to-alanine point mutation at position 234 (L234A) and a leucine-to-alanine point mutation at position 235 (L235A) in the heavy chain constant region (SEQ ID NO: 21), and were designated as 19F3H1L1(hG1DM), 19F3H2L2(hG1DM) and 19F3H2L3(hG 1DM).

The heavy chain cDNA and light chain cDNA of 19F3H1L1(hG1DM), the heavy chain cDNA and light chain cDNA of 19F3H2L2(hG1DM) and the heavy chain cDNA and light chain cDNA of 19F3H2L3(hG1DM) were separately cloned into pUC57simple (provided by Genscript) vectors to obtain pUC57simple-19F3H1(hG1DM) and pUC57simple-19F3L1; pUC57simple-19F3H2(hG 1DM), pUC57simple-19F3L2 and pUC57simple-19F3L3. Referring to the standard techniques described in Molecular Cloning: A Laboratory Manual (Second Edition), the heavy and light chain full-length genes synthesized by EcoRI&HindIII digestion were subcloned into an expression vector pcDNA3.1 through digestion by a restriction enzyme (EcoRI&HindIII) to obtain expression plasmids pcDNA3.1-19F3H1(hG1DM), pcDNA3.1-19F3L 1, pcDNA3.1-19F3H2(hG1DM), pcDNA3.1-19F3L2 and pcDNA3.1-19F3L3, and the heavy/light chain genes of the recombinant expression plasmids were further subjected to sequencing analysis. Then the designed gene combinations comprising the corresponding light and heavy chain recombinant plasmids (pcDNA3.1-19F3H1(hG1DM)/pcDNA3.1-19F3L1, pcDNA3.1-19F3H2(hG1DM)/pcDNA3.1-19F3L2, and pcDNA3.1-19F3H2(hG1DM)/pcDNA3.1-19F3L3) were separately co-transfected into 293F cells, and the culture solutions were collected and purified. After the sequences were verified, endotoxin-free expression plasmids were prepared, and were transiently transfected into HEK293 cells for antibody expression. The culture solutions were collected after 7 days, and subjected to affinity purification on a Protein A column to obtain humanized antibodies.

### Example 4: Dynamic Affinity Assay of Anti-CD73 Antibodies with C1q and FcγRIIIa

### (1) Dynamic affinity assay of anti-CD73 antibodies with C1q

The sample dilution buffer was PBS (0.02% Tween-20, 0.1% BSA, pH7.4). 50 µg/mL antibody was immobilized on the FAB2G sensor at an immobilization height of about 2.0 nm. The sensor was equilibrated in a buffer for 60 s for blocking, and the binding of the immobilized antibody on the sensor to the antigen C1q at concentrations of 0.63-10 nM (serial two-fold dilution) was assayed for 60 s. The antigen-antibody was dissociated in the buffer for 60 s. The sensor was refreshed 4 times in 10 mM glycine pH 1.7, each for 5 s. The shaking speed of the sample plate was 1000 rpm, the temperature was 30 °C and the frequency was 0.6 Hz. The data were analyzed by 1:1 model fitting to obtain affinity constants. The data acquisition software was Fortebio Data Acquisition 7.0, and the data analysis software was Fortebio Data Analysis 7.0.

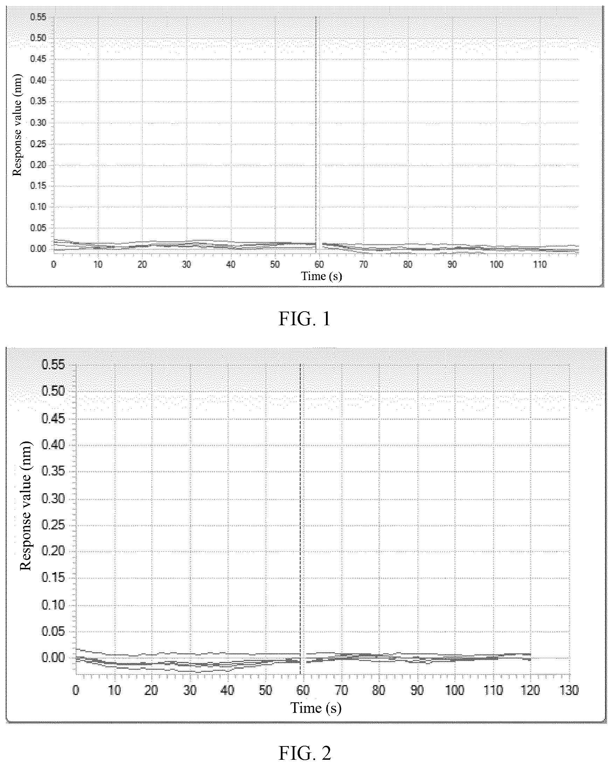

According to the results shown in Table 1 and FIGs. 1-3, neither 19F3H2L3(hG1DM) nor MEDI9447 had binding activity to C1q.

| **Sample ID** | **KD (M)** | **kon (1/Ms)** | **Standard error (kon)** | **kdis(1/s)** | **Standard error (kdis)** |
|---|---|---|---|---|---|
| 19F3H2L3(hG1DM) | N/A | N/A | N/A | N/A | N/A |
| MEDI9447 | N/A | N/A | N/A | N/A | N/A |
| Wild-type IgG1 antibody | 1.37E-09 | 5.53E+06 | 4.27E+05 | 7.58E-03 | 4.84E-04 |

| | | | | | |
|---|---|---|---|---|---|
| KD = kdis/kon | | | | | |

### (2) Dynamic affinity assay of anti-CD73 antibodies with FcyRIIIa

The sample dilution buffer was PBS (0.02% Tween-20, 0.1% BSA, pH 7.4). 0.5 µg/mL FcyRIIIa (from Sino Biological) was immobilized on the SA sensor for 120 s. The sensor was equilibrated in a buffer for 60 s, and the binding of the immobilized CD16a on the sensor to the antibodies at concentrations of 31.3-500 nM (serial two-fold dilution) was assayed for 60 s. The antibody-antigen was dissociated in the buffer for 60 s. The sensor was refreshed with 10 mM NaOH. The temperature was 30 °C and the frequency was 0.6 Hz. The data were analyzed by 1:1 model fitting to obtain affinity constants.

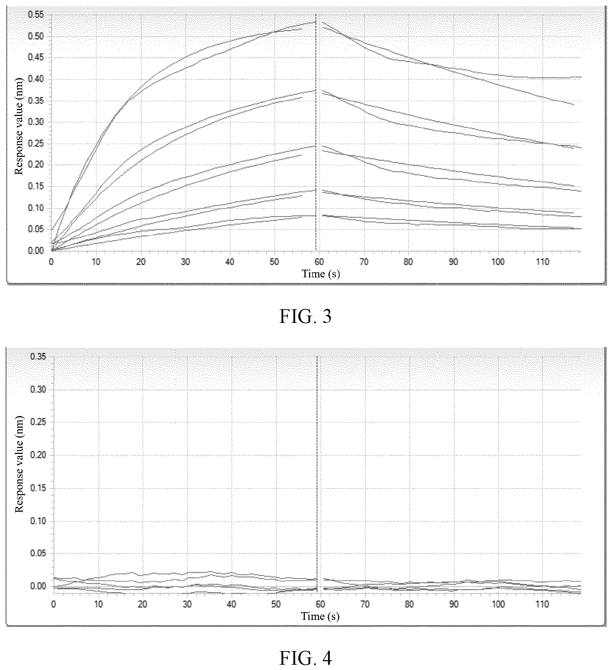

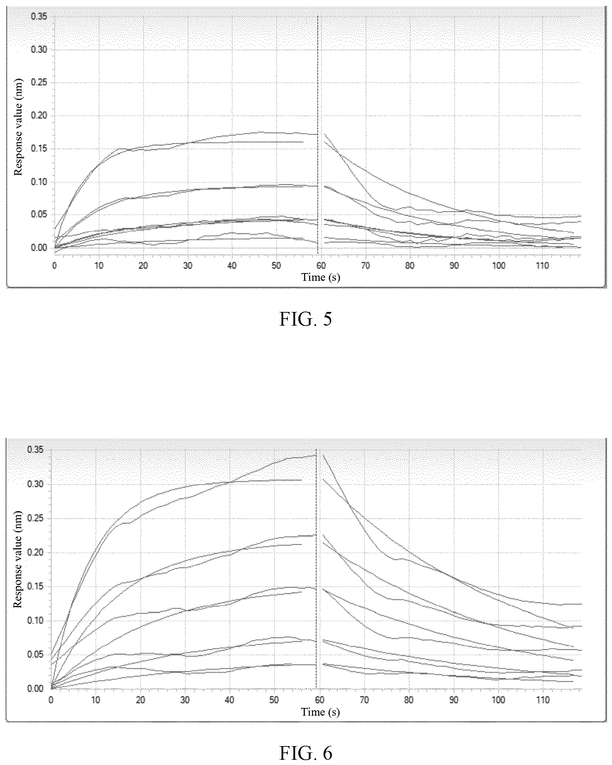

According to the results shown in Table 2 and FIGs. 4-6, 19F3H2L3(hG1DM) did not bind to FcyRIIIa, whereas MEDI9447 had binding activity to FcyRIIIa.

| **Sample ID** | **KD (M)** | **kon (1/Ms)** | **Standard error (kon)** | **kdis(1/s)** | **Standard error (kdis)** |
|---|---|---|---|---|---|
| 19F3H2L3(hG1DM) | N/A | N/A | N/A | N/A | N/A |
| MEDI9447 | 1.41E-07 | 2.43E+05 | 4.36E+04 | 3.42E-02 | 2.08E-03 |
| Wild-type IgG1 antibody | 1.25E-07 | 1.76E+05 | 2.22E+04 | 2.20E-02 | 1.11E-03 |

| | | | | | |
|---|---|---|---|---|---|
| KD = kdis/kon | | | | | |

### Example 5. Detection of Inhibition of Anti-CD73 Antibody on Enzyme Activity of CD73 Endogenously Expressed in Cells

The experimental procedures were as follows. MDA-MB-231 cells (from ATCC, HTB-26) in logarithmic phase in good condition were taken, resuspended in a serum-free RPMI-1640 culture solution, and then counted. The MDA-MB-231 cells were seeded into a 96-well plate at 3 × 10⁴ cells/100 µL/well. The antibody was diluted with the serum-free RPMI-1640 culture solution at an initial concentration of 200 µg/mL (serial 2.5-fold dilution). The antibody was added to the 96-well plate at 50 µL/well, and the plate was incubated at 37 °C for 1 h. After 1 h, 50 µL of RPMI-1640-diluted 600 µM AMP was added to each well. After 3 hours, 25 µL of cell culture supernatant was taken and transferred to a new 96-well plate, and 25 µL of 100 µM ATP was added to each well. 50 µL of CTG (CellTiter-Glo^{®} One Solution Assay, promega, Cat. No. G8461) color developing solution was added to each well for color development, and data were read by a multi-label microplate tester (PerkinElmer, Cat No. 2140-0020). The isotype control antibody and the CD73-specific inhibitor APCP were taken as negative and positive controls, respectively.

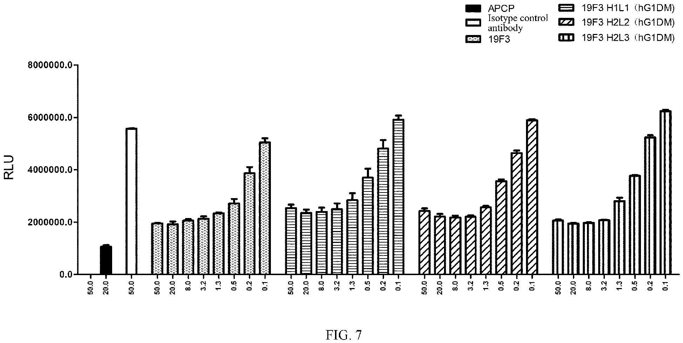

The experimental results were as follows: as shown in FIG. 7, 19F3, 19F3H2L3(hG1DM), 19F3H2L3(hG1DM) and 19F3H2L3(hG1DM) all showed dose-dependent inhibition of the activity of the endogenously-expressed CD73 enzyme catalyzing AMP to adenosine A in MDA-MB-231, thereby dose-dependently reducing the mean fluorescence intensity RLU produced.

The above experimental results showed that the added AMP could be catalyzed by CD73 enzyme expressed endogenously on the cell surface by MDA-MB-231 and then converted into adenosine under the condition of no CD73 antibody treatment, so that the inhibition of luciferase activity was relieved. However, after addition of the antibody, as CD73 was bound by the antibody, its enzymatic activity was reduced, so that AMP could not be converted into adenosine. It is suggested that the anti-CD73 antibody effectively inhibits the enzyme activity reaction of CD73 in a non-substrate competition mode and reduces the production of adenosine.

### Example 6: Pharmacodynamic Evaluation of Anti-CD73 Specific Antibody in Combination with Anti-PD-1-Anti-VEGFA Bispecific Antibody in Mouse Tumor Cell Subcutaneous Xenograft Model

In order to determine the anti-tumor activity *in vivo* of the anti-CD73 specific antibody 19F3H2L3(hG1DM) in combination with the anti-PD-1-anti-VEGFA bispecific antibody VP101(hG1DM), MC38-hPDL1/hCD73 cells (purchased from GemPharmatech Co., Ltd.) were first inoculated subcutaneously into female B6-hPD1/hPDL1/hCD73 mice aged 6.7-7.7 weeks (purchased from GemPharmatech Co., Ltd.), and when the mean tumor volume reached 80-120 mm³, the mice were randomly divided into 4 groups of 6 mice per group based on tumor volume. The day of grouping was defined as D0, and administration was started on the day of grouping D0. The administration mode of the combined administration group was as follows: the drugs were formulated separately, and the anti-PD-1-anti-VEGFA bispecific antibody VP101(hG1DM) was administered first, and after 2 h the anti-CD73 specific antibody 19F3H2L3(hG1DM) was administered. The modeling and specific regimen are shown in Table 3. After the administration, the length and width of tumors in each group were measured, and the tumor volume was calculated. Table 3: Regimens for the combination of the 19F3H2L3(hG1DM) antibody and the anti-PD-1-anti-VEGFA bispecific VP101(hG1DM) for the treatment of the MC38-hPDL1/hCD73 grafted tumor B6-hPD1/hPDL1/hCD73 mouse model

| Grouping | Number | Grafting | Administration |
|---|---|---|---|
| Isotype control 30 mg/kg | 6 | MC38-hPDL1/hCD73 was injected subcutaneously to B6-hPD1/hPDL1/hCD73 mice at 2 × 10⁶ cells. | Isotype control antibody (produced by Akeso Biopharma, Inc., Batch No. 20200704) was injected intraperitoneally at 30 mg/kg twice per week for 3 weeks. |
| 19F3H2L3 | 6 | | 19F3H2L3(hGlDM) (produced by |
| (hG1DM) 30mg/kg | | | Akeso Biopharma, Inc., Batch No. A119C20200701) was injected intraperitoneally at 30 mg/kg twice per week for 3 weeks. |
| VP101 (hG1DM) 0.5 mg/kg | 6 | | VP101(hG1DM) (produced by Akeso Biopharma, Inc., Batch No. B112C20200502) was injected intraperitoneally at 0.5 mg/kg twice per week for 3 weeks. |
| 19F3H2L3 (hG1DM) 30mg/kg, VP101 (hG1DM) 0.5 mg/kg | 6 | | 19F3H2L3(hGIDM) was injected intraperitoneally at 30 mg/kg twice per week for 3 weeks; VP101(hG1DM) was injected intraperitoneally at 0.5 mg/kg twice per week for 3 weeks. |

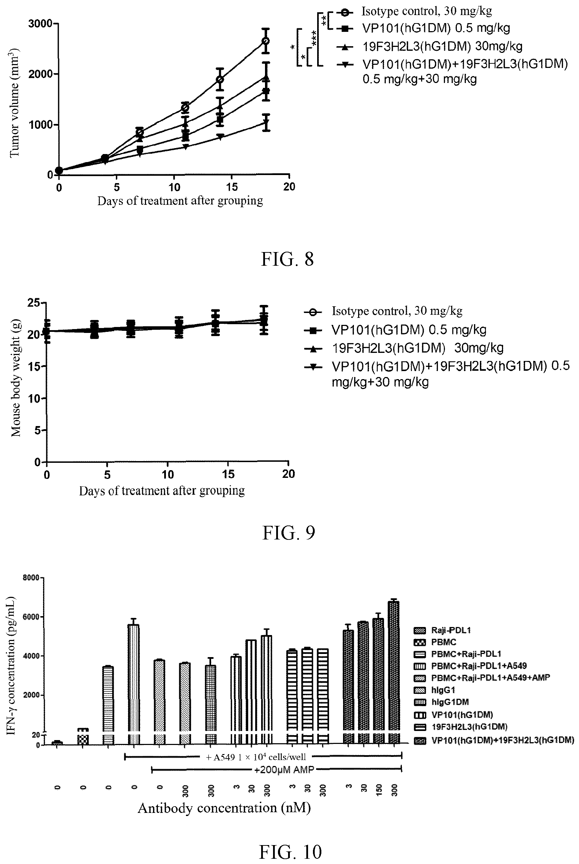

The results are shown in FIG. 8. The results showed that compared to the isotype control antibody, both the 19F3H2L3(hG1DM) antibody and the anti-PD-1-anti-VEGFA bispecific antibody VP101(hG1DM) could effectively inhibit the growth of mouse tumors, and the combination group of 19F3H2L3(hG1DM) and VP101(hG1DM) showed combined anti-tumor efficacy on the model, and the combination group was superior to the single-use group of the tested drugs in inhibiting tumors.

In addition, as shown in FIG. 9, the tumor-bearing mice were well resistant to the single use and combination use of the tested drugs 19F3H2L3(hG1DM) and VP101(hG1DM), and each group had no effect on the body weight of the tumor-bearing mice.

### Example 7: Detection of Promotion of Cytokine Secretion by Anti-CD73 Specific Antibody in Combination with Anti-PD-1-anti-VEGFA Bispecific Antibody Using Mixed Lymphocyte Reaction

In the present invention, the GenBank accession number of PDL1 is NP_054862.1, and PDL1FL represents the full length of PDL1 gene.

Human PD-L1 overexpression lentiviral vector plenti6.3/V5-PDL1FL-BSD (plenti6.3/V5-BSD purchased from Invitrogen, product number: K5315-20) infected Raji cells after virus packaging, and Raji-PDL1 cell lines were obtained after BSD (Blasticidin, Gibco product number: R210-01) drug screening.

Normal human PBMCs were obtained by separation according to the operating instructions of Ficoll-Paque^{™} Plus. Two days prior to the experiment, PBMCs were thawed and cultured in a complete medium (1640 + 10% FBS) at 37 °C in a 5% carbon dioxide incubator. After 2 h, when the PBMC status recovered, SEB (with a final concentration of 0.5 µg/mL, Toxin technology, Cat No. BT202) was added to stimulate cells for two days. On the day of the experiment, Raji-PDL1 cells (constructed by Akeso Biopharma, Inc.) were collected, centrifuged, resuspended (in an analysis medium 1640 + 10% FBS), counted and adjusted to a density of 2 × 10⁶ cells/mL. MMC (Mito-mycin C, with a final concentration of 2 µg/mL, Stressmarq, Cat No. SIH-246-10MG) was added, and the mixture was treated at 37 °C for 1 h in a 5% carbon dioxide incubator. A549 lung cancer cells (purchased from Chinese academy of sciences) were routinely collected, centrifuged, resuspended and counted. PBMCs after two days of SEB stimulation and Raji-PDL1 cells after MMC treatment were collected, and seeding was performed on a 96-well U-shaped plate (Corning, Cat No. 3799) at 1 × 10⁵ cells/well for PBMC, 1 × 10⁵ cells/well for Raji-PDL1, and 1 × 10⁴ cells/well for A549, respectively; AMP (adenosine-5'-monophosphate, TCI, Cat No. A0158) and the antibody were added according to the experimental design, and negative controls (PBMC + Raji-PDL1 + A549 + AMP) and isotype controls (hIgG1 and hIgGDM) were set. The cells were incubated for a total of 3 days (the final volume of the system was 200 µL). After 3 days, the cells were centrifuged at 250 × g for 5 min (Beckman centrifuge), and cell supernatants were collected. IFN-γ contents were detected using a Dakewe kit.

The results are shown in FIG. 10. The results show that: compared to the isotype control antibody, the anti-PD-1-anti-VEGFA bispecific antibody VP101(hG1DM) can effectively promote secretion of cytokine IFN-γ in a mixed lymphocyte system, and the combination group of 19F3H2L3(hG1DM) + VP101(hG1DM) shows combined anti-tumor efficacy. The combination group is superior to the monotherapy group in promoting secretion of cytokine IFN-γ, and the combined activity of 150 nM 19F3H2L3(hG1DM) + VP101(hG1DM) is superior to that of 300 nM 19F3H2L3(hG1DM) or VP101(hG1DM) monotherapy.

### SEQUENCE LISTING

The nucleic acid sequence of the heavy chain variable region of 19F3: (SEQ ID NO: 1) 
GAGGTGCAGCTGCAGCAGTCCGGACCAGAGCTGGTGAAGCCTGGC
GCCTCCATGCGGATGTCTTGTAAGGCCTCTGGCTACAGCTTCACCGG
CTATACAATGAACTGGGTGAAGCAGTCTCACGGCAAGAATCTGGAG
TGGATCGGCCTGATCAACCCTTACAATGCCGGCACCAGCTATAACCA
GAAGTTTAAGGGCAAGGCCACCCTGACAGTGGACAAGAGCTCCTCT
ACCGCCTACATGGAGCTGCTGTCCCTGACATCTGAGGATAGCGCCGT
GTACTATTGCGCCCGGTCCGAGTACAGATATGGCGGCGACTACTTTG
ATTATTGGGGCCAGGGCACCACACTGACAGTGAGCTCC
The amino acid sequence of the heavy chain variable region of 19F3: (SEQ ID NO: 2) 
EVQLQQSGPELVKPGASMRMSCKASGYSFTGYTMNWVKQSHGKNLE
WIGLINPYNAGTSYNQKFKGKATLTVDKSSSTAYMELLSLTSEDSAVYY
CARSEYRYGGDYFDYWGQGTTLTVSS
The nucleic acid sequence of the light chain variable region of 19F3: (SEQ ID NO: 3) 
GACATCGTGATGACCCAGTCTCCAAGCTCCCTGGCAATGAGCGTGG
GACAGAAGGTGACAATGTCTTGTAAGTCTAGCCAGAGCCTGCTGAA
CTCCTCTAATCAGAAGAACTACCTGGCCTGGTATCAGCAGAAGCCA
GGCCAGTCTCCCAAGCTGCTGGTGTACTTTGCCAGCACCAGGGAGT
CCGGAGTGCCTGACAGATTCATCGGCTCCGGCTCTGGCACAGACTT
CACCCTGACAATCAGCTCCGTGCAGGCAGAGGACCTGGCAGATTAT
TTCTGCCAGCAGCACTACGACACCCCTTATACATTTGGCGGCGGCAC
CAAGCTGGAGATCAAG
The amino acid sequence of the light chain variable region of 19F3: (SEQ ID NO: 4) 
DIVMTQSPSSLAMSVGQKVTMSCKSSQSLLNSSNQKNYLAWYQQKPG
QSPKLLVYFASTRESGVPDRFIGSGSGTDFTLTISSVQAEDLADYFCQQH
YDTPYTFGGGTKLEIK
The nucleic acid sequence of the heavy chain variable region of 19F3H1L1(hG1DM): (SEQ ID NO: 5) 
CAGGTGCAGCTGCAGCAGTCTGGAGCAGAGGTGGTGAAGCCAGGA
GCCTCTATGAAGATGAGCTGTAAGGCCAGCGGCTACTCCTTCACCGG
CTATACAATGAACTGGGTGAAGCAGGCCCACGGCCAGAATCTGGAG
TGGATCGGCCTGATCAACCCTTACAATGCCGGCACCTCTTATAACCA
GAAGTTTCAGGGCAAGGCCACCCTGACAGTGGACAAGTCCACCTCT
ACAGCCTACATGGAGCTGAGCTCCCTGCGGAGCGAGGATACAGCCG
TGTACTATTGCGCCCGGTCCGAGTACAGATATGGCGGCGACTACTTT
GATTATTGGGGCCAGGGCACCACACTGACCGTGTCTAGC
The amino acid sequence of the heavy chain variable region of 19F3H1L1(hG1DM): (SEQ ID NO: 6) 
QVQLQQSGAEVVKPGASMKMSCKASGYSFTGYTMNWVKQAHGQNL
EWIGLINPYNAGTSYNQKFQGKATLTVDKSTSTAYMELSSLRSEDTAVY
YCARSEYRYGGDYFDYWGQGTTLTVSS
The nucleic acid sequence of the light chain variable region of 19F3H1L1(hG1DM): (SEQ ID NO: 7) 
GACATCGTGATGACCCAGTCCCCAAGCTCCCTGGCAATGTCTGTGG
GAGAGAGGGTGACAATGTCCTGTAAGTCTAGCCAGTCTCTGCTGAA
CTCCTCTAATCAGAAGAACTACCTGGCCTGGTATCAGCAGAAGCCC
GGCCAGGCCCCTAAGCTGCTGGTGTACTTTGCCTCTACCAGGGAGA
GCGGAGTGCCAGACAGATTCTCTGGCAGCGGCTCCGGCACAGACTT
CACCCTGACAATCAGCTCCGTGCAGGCAGAGGACCTGGCAGATTAT
TTCTGCCAGCAGCACTACGATACCCCCTATACATTTGGCGGCGGCAC
CAAGCTGGAGATCAAG
The amino acid sequence of the light chain variable region of 19F3H1L1(hG1DM): (SEQ ID NO: 8) 
DIVMTQSPSSLAMSVGERVTMSCKSSQSLLNSSNQKNYLAWYQQKPG
QAPKLLVYFASTRESGVPDRFSGSGSGTDFTLTISSVQAEDLADYFCQQ
HYDTPYTFGGGTKLEIK
The nucleic acid sequences of the heavy chain variable regions of 19F3H2L2(hG1DM) and 19F3H2L3(hG1DM): (SEQ ID NO: 9) 
CAGGTGCAGCTGGTGCAGTCTGGAGCAGAGGTGGTGAAGCCAGGA
GCCTCTGTGAAGGTGAGCTGTAAGGCCAGCGGCTACTCCTTCACCG
GCTATACAATGAACTGGGTGAGGCAGGCACCAGGACAGAATCTGGA
GTGGATCGGCCTGATCAACCCTTACAATGCCGGCACCTCTTATAACC
AGAAGTTTCAGGGCAAGGTGACCCTGACAGTGGACAAGTCCACCT
CTACAGCCTACATGGAGCTGAGCTCCCTGCGGAGCGAGGATACAGC
CGTGTACTATTGCGCCCGGTCCGAGTACAGATATGGCGGCGACTACT
TTGATTATTGGGGCCAGGGCACCACACTGACCGTGTCTAGC
The amino acid sequences of the heavy chain variable regions of 19F3H2L2(hG1DM) and 19F3H2L3(hG1DM): (SEQ ID NO: 10) 
QVQLVQSGAEVVKPGASVKVSCKASGYSFTGYTMNWVRQAPGQNLE
WIGLINPYNAGTSYNQKFQGKVTLTVDKSTSTAYMELSSLRSEDTAVY
YCARSEYRYGGDYFDYWGQGTTLTVSS
The nucleic acid sequence of the light chain variable region of 19F3H2L2(hG1DM): (SEQ ID NO: 11) 
GACATCGTGATGACCCAGTCCCCAAGCTCCCTGGCCGTGTCTGTGG
GAGAGCGGGTGACAATCTCCTGTAAGTCTAGCCAGTCTCTGCTGAA
CTCCTCTAATCAGAAGAACTACCTGGCCTGGTATCAGCAGAAGCCC
GGCCAGGCCCCTAAGCTGCTGATCTACTTCGCCTCTACCAGGGAGA
GCGGAGTGCCAGACAGATTCTCTGGCAGCGGCTCCGGCACAGACTT
 
CACCCTGACAATCAGCTCCGTGCAGGCAGAGGACGTGGCAGATTAC
TATTGCCAGCAGCACTACGATACCCCCTATACATTTGGCGGCGGCAC
CAAGCTGGAGATCAAG
The amino acid sequence of the light chain variable region of 19F3H2L2(hG1DM): (SEQ ID NO: 12) 
DIVMTQSPSSLAVSVGERVTISCKSSQSLLNSSNQKNYLAWYQQKPGQ
APKLLIYFASTRESGVPDRFSGSGSGTDFTLTISSVQAEDVADYYCQQH
YDTPYTFGGGTKLEIK
The nucleic acid sequence of the light chain variable region of 19F3H2L3(hG1DM): (SEQ ID NO: 13) 
GACATCGTGATGACCCAGTCCCCAAGCTCCCTGGCCGTGTCTGTGG
GAGAGCGGGTGACAATCTCCTGTAAGTCTAGCCAGTCTCTGCTGAA
CTCCTCTAATCAGAAGAACTACCTGGCCTGGTATCAGCAGAAGCCC
GGCCAGGCCCCTAAGCTGCTGATCTACTTCGCCTCTACCAGGGAGA
GCGGAGTGCCAGACAGATTCTCTGGCAGCGGCTCCGGCACAGACTT
CACCCTGACAATCAGCTCCCTGCAGGCAGAGGACGTGGCCGTGTAC
TATTGCCAGCAGCACTACGATACCCCCTATACATTTGGCGGCGGCAC
CAAGCTGGAGATCAAG
The amino acid sequence of the light chain variable region of 19F3H2L3(hG1DM): (SEQ ID NO: 14) 
DIVMTQSPSSLAVSVGERVTISCKSSQSLLNSSNQKNYLAWYQQKPGQ
APKLLIYFASTRESGVPDRFSGSGSGTDFTLTISSLQAEDVAVYYCQQHY
DTPYTFGGGTKLEIK
The CDRs of 19F3 and 19F3H1L1(hG1DM), 19F3H2L2(hG1DM), and 19F3H2L3(hG1DM)
   HCDR1: GYSFTGYT (SEQ ID NO: 15)
   HCDR2: INPYNAGT (SEQ ID NO: 16)
   HCDR3: ARSEYRYGGDYFDY (SEQ ID NO: 17)
   LCDR1: QSLLNSSNQKNY (SEQ ID NO: 18)
   LCDR2: FAS (SEQ ID NO: 19)
   LCDR3: QQHYDTPYT (SEQ ID NO: 20)
The sequences of heavy chain constant regions (330 aa, mutation sites underlined) of 19F3H1L1(hG1DM), 19F3H2L2(hG1DM) and 19F3H2L3 (hG1DM): 
ASTKGPSVFPLAPSSKSTSGGTAALGCLVKDYFPEPVTVSWNSGALTSG
VHTFPAVLQSSGLYSLSSVVTVPSSSLGTQTYICNVNHKPSNTKVDKKV
EPKSCDKTHTCPPCPAPEAAGGPSVFLFPPKPKDTLMISRTPEVTCVVV
DVSHEDPEVKFNWYVDGVEVHNAKTKPREEQYNSTYRVVSVLTVLH
QDWLNGKEYKCKVSNKALPAPIEKTISKAKGQPREPQVYTLPPSRDEL
TKNQVSLTCLVKGFYPSDIAVEWESNGQPENNYKTTPPVLDSDGSFFLY
SKLTVDKSRWQQGNVFSCS VMHEALHNHYTQKSLSLSPGK (SEQ ID
NO: 21)
The sequences of the light chain constant regions (107 aa) of 19F3H1L 1 (hG 1DM), 19F3H2L2(hG1DM) and 19F3H2L3(hG1DM): 


 (SEQ ID NO: 22)
The amino acid sequence of the heavy chain of VP101(hG1DM) 
EVQLVESGGGLVQPGGSLRLSCAASGYTFTNYGMNWVRQAPGKGLE
WVGWINTYTGEPTYAADFKRRFTFSLDTSKSTAYLQMNSLRAEDTAVY
YCAKYPHYYGSSHWYFDVWGQGTLVTVSSASTKGPSVFPLAPSSKSTS
GGTAALGCLVKDYFPEPVTVSWNSGALTSGVHTFPAVLQSSGLYSLSSV
VTVPSSSLGTQTYICNVNHKPSNTKVDKKVEPKSCDKTHTCPPCPAPE
AAGGPSVFLFPPKPKDTLMISRTPEVTCVVVDVSHEDPEVKFNWYVDG
VEVHNAKTKPREEQYNSTYRVVSVLTVLHQDWLNGKEYKCKVSNKA
 
LPAPIEKTISKAKGQPREPQVYTLPPSRDELTKNQVSLTCLVKGFYPSDI
AVEWESNGQPENNYKTTPPVLDSDGSFFLYSKLTVDKSRWQQGNVFSC
SVMHEALHNHYTQKSLSLSPGKGGGGSGGGGSGGGGSGGGGSEVQL
VESGGGLVQPGGSLRLSCAASGFAFSSYDMSWVRQAPGKGLDWVATI
SGGGRYTYYPDSVKGRFTISRDNSKNNLYLQMNSLRAEDTALYYCAN
RYGEAWFAYWGQGTLVTVSSGGGGSGGGGSGGGGSGGGGSDIQMTQ
SPSSMSASVGDRVTFTCRASQDINTYLSWFQQKPGKSPKTLIYRANRLV
SGVPSRFSGSGSGQDYTLTISSLQPEDMATYYCLQYDEFPLTFGAGTKL
ELKR (SEQ ID NO: 23)
The nucleic acid sequence of the heavy chain of VP101(hG1DM) 
GAGGTGCAGCTGGTCGAGTCCGGGGGGGGGCTGGTGCAGCCAGGC
GGGTCTCTGAGGCTGAGTTGCGCCGCTTCAGGGTACACCTTCACAA
ACTATGGAATGAATTGGGTGCGCCAGGCACCAGGAAAGGGACTGGA
GTGGGTCGGCTGGATCAACACTTACACCGGGGAACCTACCTATGCA
GCCGACTTTAAGCGGCGGTTCACCTTCAGCCTGGATACAAGCAAAT
CCACTGCCTACCTGCAGATGAACAGCCTGCGAGCTGAGGACACCGC
AGTCTACTATTGTGCTAAATATCCCCACTACTATGGGAGCAGCCATTG
GTATTTTGACGTGTGGGGGCAGGGGACTCTGGTGACAGTGAGCAGC
GCAAGCACCAAAGGGCCCAGCGTGTTTCCTCTCGCCCCCTCCTCCA
AAAGCACCAGCGGAGGAACCGCTGCTCTCGGATGTCTGGTGAAGG
ACTACTTCCCTGAACCCGTCACCGTGAGCTGGAATAGCGGCGCTCT
GACAAGCGGAGTCCATACATTCCCTGCTGTGCTGCAAAGCAGCGGA
CTCTATTCCCTGTCCAGCGTCGTCACAGTGCCCAGCAGCAGCCTGGG
CACCCAGACCTACATCTGTAACGTCAACCACAAGCCCTCCAACACC
AAGGTGGACAAGAAAGTGGAGCCCAAATCCTGCGACAAGACACAC
ACCTGTCCCCCCTGTCCTGCTCCCGAAGCTGCTGGAGGCCCTAGCGT
CTTCCTCTTTCCTCCCAAACCCAAGGACACCCTCATGATCAGCAGAA
CCCCTGAAGTCACCTGTGTCGTCGTGGATGTCAGCCATGAGGACCC
CGAGGTGAAATTCAACTGGTATGTCGATGGCGTCGAGGTGCACAAC
GCCAAAACCAAGCCCAGGGAGGAACAGTACAACTCCACCTACAGG
 
GTGGTGTCCGTGCTGACAGTCCTCCACCAGGACTGGCTGAACGGCA
AGGAGTACAAGTGCAAGGTGTCCAACAAGGCTCTCCCTGCCCCCAT
TGAGAAGACCATCAGCAAGGCCAAAGGCCAACCCAGGGAGCCCCA
GGTCTATACACTGCCTCCCTCCAGGGACGAACTCACCAAGAACCAG
GTGTCCCTGACCTGCCTGGTCAAGGGCTTTTATCCCAGCGACATCGC
CGTCGAGTGGGAGTCCAACGGACAGCCCGAGAATAACTACAAGAC
CACCCCTCCTGTCCTCGACTCCGACGGCTCCTTCTTCCTGTACAGCA
AACTGACCGTCGATAAATCTAGGTGGCAGCAGGGCAACGTGTTCTC
TTGTTCCGTGATGCATGAAGCACTGCACAACCATTATACCCAGAAGT
CTCTGAGCCTGTCCCCCGGCAAGGGCGGCGGCTCTGGAGGAG
GAGGCAGCGGCGGAGGAGGCTCCGGAGGCGGCGGCTCTGAGGTGC
AGCTGGTGGAGTCTGGAGGAGGACTGGTGCAGCCTGGAGGCTCCC
TGAGGCTGTCTTGCGCAGCAAGCGGATTCGCCTTTAGCTCCTACGAC
ATGAGCTGGGTGCGGCAGGCACCTGGCAAGGGTCTGGATTGGGTGG
CAACCATCAGCGGAGGCGGCAGATACACATACTATCCCGACTCCGTG
AAGGGCAGGTTCACCATCTCCCGCGATAACTCTAAGAACAATCTGTA
TCTGCAGATGAACAGCCTGAGGGCCGAGGACACAGCCCTGTACTAT
TGCGCCAACCGCTACGGCGAGGCCTGGTTTGCCTATTGGGGCCAGG
GCACCCTGGTGACAGTGTCTAGCGGCGGCGGCGGCAGCGGCGGCG
GCGGCTCCGGAGGAGGCGGCTCTGGCGGCGGCGGCAGCGATATCCA
GATGACCCAGTCCCCCTCCTCTATGTCTGCCAGCGTGGGCGACCGGG
TGACCTTCACATGTAGAGCCTCCCAGGATATCAACACCTACCTGTCT
TGGTTTCAGCAGAAGCCCGGCAAGAGCCCTAAGACACTGATCTATC
GGGCCAATAGACTGGTGAGCGGAGTGCCTTCCCGGTTCTCCGGCTC
TGGCAGCGGACAGGACTATACCCTGACAATCAGCTCCCTGCAGCCA
GAGGATATGGCCACATACTATTGCCTGCAGTATGACGAGTTCCCCCT
GACCTTCGGGGCTGGCACTAAGCTGGAGCTGAAAAGA (SEQ ID NO:
24)
The amino acid sequence of the light chain of VP101(hG1DM) 
DIQMTQSPSSLSASVGDRVTITCSASQDISNYLNWYQQKPGKAPKVLIY
FTSSLHSGVPSRFSGSGSGTDFTLTISSLQPEDFATYYCQQYSTVPWTFG
QGTKVEIKRTVAAPSVFIFPPSDEQLKSGTASVVCLLNNFYPREAKVQW
KVDNALQSGNSQESVTEQDSKDSTYSLSSTLTLSKADYEKHKVYACEV
THQGLSSPVTKSFNRGEC (SEQ ID NO: 25)
The nucleic acid sequence of the light chain of VP101(hG1DM) 
GATATTCAGATGACTCAGAGCCCCTCCTCCCTGTCCGCCTCTGTGGG
CGACAGGGTCACCATCACATGCAGTGCTTCACAGGATATTTCCAACT
ACCTGAATTGGTATCAGCAGAAGCCAGGAAAAGCACCCAAGGTGCT
GATCTACTTCACTAGCTCCCTGCACTCAGGAGTGCCAAGCCGGTTCA
GCGGATCCGGATCTGGAACCGACTTTACTCTGACCATTTCTAGTCTG
CAGCCTGAGGATTTCGCTACATACTATTGCCAGCAGTATTCTACCGTG
CCATGGACATTTGGCCAGGGGACTAAAGTCGAGATCAAGCGGACCG
TGGCCGCTCCCAGTGTCTTCATTTTTCCCCCTAGCGACGAACAGCTG
AAATCCGGGACAGCCTCTGTGGTCTGTCTGCTGAACAACTTCTACCC
TAGAGAGGCAAAAGTGCAGTGGAAGGTCGATAACGCCCTGCAGAG
TGGCAATTCACAGGAGAGCGTGACAGAACAGGACTCCAAAGATTCT
ACTTATAGTCTGTCAAGCACACTGACTCTGAGCAAGGCTGACTACG
AAAAGCATAAAGTGTATGCATGTGAGGTCACCCACCAGGGGCTGAG
CAGTCCAGTCACCAAGTCATTCAACAGAGGCGAGTGC (SEQ ID NO:
26)
The amino acid sequence of bevacizumab heavy chain variable region (Bevacizumab-Hv): (123 aa) 


 (SEQ ID NO: 27)
The nucleic acid sequence encoding bevacizumab heavy chain variable region: (369 bp) 
GAGGTGCAGCTGGTCGAGTCCGGGGGGGGGCTGGTGCAGCCAGGC
GGGTCTCTGAGGCTGAGTTGCGCCGCTTCAGGGTACACCTTCACAA
ACTATGGAATGAATTGGGTGCGCCAGGCACCAGGAAAGGGACTGGA
GTGGGTCGGCTGGATCAACACTTACACCGGGGAACCTACCTATGCA
GCCGACTTTAAGCGGCGGTTCACCTTCAGCCTGGATACAAGCAAAT
CCACTGCCTACCTGCAGATGAACAGCCTGCGAGCTGAGGACACCGC
AGTCTACTATTGTGCTAAATATCCCCACTACTATGGGAGCAGCCATTG
GTATTTTGACGTGTGGGGGCAGGGGACTCTGGTGACAGTGAGCAGC
(SEQ ID NO: 28)
The amino acid sequence of bevacizumab light chain variable region (Bevacizumab-Lv): (107 aa) 


 (SEQ ID NO: 29)
The nucleic acid sequence encoding bevacizumab light chain variable region: (321 bp) 
GATATTCAGATGACTCAGAGCCCCTCCTCCCTGTCCGCCTCTGTGGG
CGACAGGGTCACCATCACATGCAGTGCTTCACAGGATATTTCCAACT
ACCTGAATTGGTATCAGCAGAAGCCAGGAAAAGCACCCAAGGTGCT
GATCTACTTCACTAGCTCCCTGCACTCAGGAGTGCCAAGCCGGTTCA
GCGGATCCGGATCTGGAACCGACTTTACTCTGACCATTTCTAGTCTG
CAGCCTGAGGATTTCGCTACATACTATTGCCAGCAGTATTCTACCGTG
CCATGGACATTTGGCCAGGGGACTAAAGTCGAGATCAAG (SEQ ID
NO: 30)
The amino acid sequences of the 3 CDRs of the heavy chain variable region of Bevacizumab are as follows:
   HCDR1: GYTFTNYG (SEQ ID NO: 31)
   HCDR2: INTYTGEP (SEQ ID NO: 32)
   HCDR3: AKYPHYYGSSHWYFDV (SEQ ID NO: 33)
The amino acid sequences of the 3 CDRs of the light chain variable region of Bevacizumab are as follows:
   LCDR1: QDISNY (SEQ ID NO: 34)
   LCDR2: FTS (SEQ ID NO: 35)
   LCDR3: QQYSTVPWT (SEQ ID NO: 36)
The amino acid sequence of the heavy chain variable region of 14C12H1L1(M) is set forth in SEQ ID NO: 37. 
EVQLVESGGGLVQPGGSLRLSCAASGFAFSSYDMSWVRQAPGKGLDW
VATISGGGRYTYYPDSVKGRFTISRDNSKNNLYLQMNSLRAEDTALYY
CANRYGEAWFAYWGQGTLVTVSS (SEQ ID NO: 37)
The nucleic acid sequence encoding the heavy chain variable region of 14C12H1L1: (354 bp) 
GAAGTGCAGCTGGTCGAGTCTGGGGGAGGGCTGGTGCAGCCCGGC
GGGTCACTGCGACTGAGCTGCGCAGCTTCCGGATTCGCCTTTAGCTC
CTACGACATGTCCTGGGTGCGACAGGCACCAGGAAAGGGACTGGAT
TGGGTCGCTACTATCTCAGGAGGCGGGAGATACACCTACTATCCTGA
CAGCGTCAAGGGCCGGTTCACAATCTCTAGAGATAACAGTAAGAAC
AATCTGTATCTGCAGATGAACAGCCTGAGGGCTGAGGACACCGCAC
TGTACTATTGTGCCAACCGCTACGGGGAAGCATGGTTTGCCTATTGG
GGGCAGGGAACCCTGGTGACAGTCTCTAGT (SEQ ID NO: 38)
The amino acid sequence of the light chain variable region of 14C12H1L1(M) 
DIQMTQSPSSMSASVGDRVTFTCRASQDINTYLSWFQQKPGKSPKTLIY
RANRLVSGVPSRFSGSGSGQDYTLTISSLQPEDMATYYCLQYDEFPLTF
GAGTKLELKR (SEQ ID NO: 39)
The nucleic acid sequence of the light chain variable region 14C12L1(M) of 14C12H1L1(M): 
GATATCCAGATGACCCAGTCCCCCTCCTCTATGTCTGCCAGCGTGGG
CGACCGGGTGACCTTCACATGTAGAGCCTCCCAGGATATCAACACCT
ACCTGTCTTGGTTTCAGCAGAAGCCCGGCAAGAGCCCTAAGACACT
GATCTATCGGGCCAATAGACTGGTGAGCGGAGTGCCTTCCCGGTTCT
CCGGCTCTGGCAGCGGACAGGACTATACCCTGACAATCAGCTCCCT
GCAGCCAGAGGATATGGCCACATACTATTGCCTGCAGTATGACGAGT
TCCCCCTGACCTTCGGGGCTGGCACTAAGCTGGAGCTGAAAAGA
(SEQ ID NO: 40)
The CDRs of the heavy chain variable region of 14C12H1L1(M):
   HCDR1: GFAFSSYD (SEQ ID NO: 41)
   HCDR2: ISGGGRYT (SEQ ID NO: 42)
   HCDR3: ANRYGEAWFAY (SEQ ID NO: 43)
The CDRs of the light chain variable region of 14C12H1L1(M):
   LCDR1: QDINTY (SEQ ID NO: 44)
   LCDR2: RAN (SEQ ID NO: 45)
   LCDR3: LQYDEFPLT (SEQ ID NO: 46)
The amino acid sequence of the first linker fragment
   GGGGSGGGGSGGGGS (SEQ ID NO: 47)
The amino acid sequence of the second linker fragment
   GGGGSGGGGSGGGGSGGGGS (SEQ ID NO: 48)

## Claims

1. A pharmaceutical composition, comprising an anti-CD73 (e.g., human CD73) antibody or an antigen-binding fragment thereof, and an anti-PD-1-anti-VEGFA bispecific antibody or an antigen-binding fragment thereof, optionally, the pharmaceutical composition further comprising a pharmaceutically acceptable carrier and/or excipient,
wherein the anti-CD73 antibody comprises:
HCDR1, HCDR2 and HCDR3 contained in a heavy chain variable region set forth in SEQ ID NO: 2; and LCDR1, LCDR2 and LCDR3 contained in a light chain variable region set forth in SEQ ID NO: 4;
preferably, according to an IMGT numbering system, the anti-CD73 antibody comprises:
HCDR1 comprising or consisting of an amino acid sequence set forth in SEQ ID NO: 15, or a sequence having at least 80%, at least 81%, at least 82%, at least 83%, at least 84%, at least 85%, at least 86%, at least 87%, at least 88%, at least 89%, at least 90%, preferably at least 91%, at least 92%, at least 93%, at least 94%, at least 95%, at least 96%, at least 97%, at least 98% or at least 99% sequence identity to the sequence,
HCDR2 comprising or consisting of an amino acid sequence set forth in SEQ ID NO: 16, or a sequence having at least 80%, at least 81%, at least 82%, at least 83%, at least 84%, at least 85%, at least 86%, at least 87%, at least 88%, at least 89%, at least 90%, preferably at least 91%, at least 92%, at least 93%, at least 94%, at least 95%, at least 96%, at least 97%, at least 98% or at least 99% sequence identity to the sequence,
HCDR3 comprising or consisting of an amino acid sequence set forth in SEQ ID NO: 17, or a sequence having at least 80%, at least 81%, at least 82%, at least 83%, at least 84%, at least 85%, at least 86%, at least 87%, at least 88%, at least 89%, at least 90%, preferably at least 91%, at least 92%, at least 93%, at least 94%, at least 95%, at least 96%, at least 97%, at least 98% or at least 99% sequence identity to the sequence,
LCDR1 comprising or consisting of an amino acid sequence set forth in SEQ ID NO: 18, or a sequence having at least 80%, at least 81%, at least 82%, at least 83%, at least 84%, at least 85%, at least 86%, at least 87%, at least 88%, at least 89%, at least 90%, preferably at least 91%, at least 92%, at least 93%, at least 94%, at least 95%, at least 96%, at least 97%, at least 98% or at least 99% sequence identity to the sequence,
LCDR2 comprising or consisting of an amino acid sequence set forth in SEQ ID NO: 19, or a sequence having at least 80%, at least 81%, at least 82%, at least 83%, at least 84%, at least 85%, at least 86%, at least 87%, at least 88%, at least 89%, at least 90%, preferably at least 91%, at least 92%, at least 93%, at least 94%, at least 95%, at least 96%, at least 97%, at least 98% or at least 99% sequence identity to the sequence, and
LCDR3 comprising or consisting of an amino acid sequence set forth in SEQ ID NO: 20, or a sequence having at least 80%, at least 81%, at least 82%, at least 83%, at least 84%, at least 85%, at least 86%, at least 87%, at least 88%, at least 89%, at least 90%, preferably at least 91%, at least 92%, at least 93%, at least 94%, at least 95%, at least 96%, at least 97%, at least 98% or at least 99% sequence identity to the sequence;
the anti-PD-1-anti-VEGFA bispecific antibody comprises:
a first protein functional region targeting PD-1, and
a second protein functional region targeting VEGFA;
wherein the first protein functional region is a single chain antibody, and the second protein functional region is an immunoglobulin; or the first protein functional region is an immunoglobulin, and the second protein functional region is a single chain antibody,
wherein,
a heavy chain variable region of the immunoglobulin comprises: HCDR1-HCDR3 contained in a heavy chain variable region having amino acid sequences set forth in SEQ ID NO: 27 (preferably HCDR1-HCDR3 set forth in SEQ ID NOs: 31-33, respectively, according to the IMGT numbering system), and a light chain variable region thereof comprises: LCDR1-LCDR3 contained in a light chain variable region having amino acid sequences set forth in SEQ ID NO: 29 (preferably LCDR1-LCDR3 set forth in SEQ ID NOs: 34-36, respectively, according to the IMGT numbering system);
a heavy chain variable region of the single chain antibody comprises:
HCDR1-HCDR3 contained in a heavy chain variable region having amino acid sequences set forth in SEQ ID NO: 37 (preferably HCDR1-HCDR3 set forth in SEQ ID NOs: 41-43, respectively, according to the IMGT numbering system), and a light chain variable region thereof comprises: LCDR1-LCDR3 contained in a light chain variable region having amino acid sequences set forth in SEQ ID NO: 39 (preferably LCDR1-LCDR3 set forth in SEQ ID NOs: 44-46, respectively, according to the IMGT numbering system);
or
a heavy chain variable region of the immunoglobulin comprises: HCDR1-HCDR3 contained in a heavy chain variable region having amino acid sequences set forth in SEQ ID NO: 37 (preferably HCDR1-HCDR3 set forth in SEQ ID NOs: 41-43, respectively, according to the IMGT numbering system), and a light chain variable region thereof comprises: LCDR1-LCDR3 contained in a light chain variable region having amino acid sequences set forth in SEQ ID NO: 39 (preferably LCDR1-LCDR3 set forth in SEQ ID NOs: 44-46, respectively, according to the IMGT numbering system);
a heavy chain variable region of the single chain antibody comprises: HCDR1-HCDR3 contained in a heavy chain variable region having amino acid sequences set forth in SEQ ID NO: 27 (preferably HCDR1-HCDR3 set forth in SEQ ID NOs: 31-33, respectively, according to the IMGT numbering system), and a light chain variable region thereof comprises: LCDR1-LCDR3 contained in a light chain variable region having amino acid sequences set forth in SEQ ID NO: 29 (preferably LCDR1-LCDR3 set forth in SEQ ID NOs: 34-36, respectively, according to the IMGT numbering system).

2. The pharmaceutical composition according to claim 1, wherein the heavy chain variable region of the anti-CD73 antibody comprises or consists of the following sequences:
SEQ ID NO: 2, SEQ ID NO: 6 or SEQ ID NO: 10, or a sequence having at least 80%, at least 81%, at least 82%, at least 83%, at least 84%, at least 85%, at least 86%, at least 87%, at least 88%, at least 89%, at least 90%, preferably at least 91%, at least 92%, at least 93%, at least 94%, at least 95%, at least 96%, at least 97%, at least 98% or at least 99% sequence identity to SEQ ID NO: 2, SEQ ID NO: 6 or SEQ ID NO: 10; and
the light chain variable region of the anti-CD73 antibody comprises or consists of the following sequences:
SEQ ID NO: 4, SEQ ID NO: 8, SEQ ID NO: 12 or SEQ ID NO: 14, or a sequence having at least 80%, at least 81%, at least 82%, at least 83%, at least 84%, at least 85%, at least 86%, at least 87%, at least 88%, at least 89%, at least 90%, preferably at least 91%, at least 92%, at least 93%, at least 94%, at least 95%, at least 96%, at least 97%, at least 98% or at least 99% sequence identity to SEQ ID NO: 4, SEQ ID NO: 8, SEQ ID NO: 12 or SEQ ID NO: 14, preferably
an amino acid sequence of the heavy chain variable region of the anti-CD73 antibody is set forth in SEQ ID NO: 2 (preferably, a nucleic acid sequence is set forth in SEQ ID NO: 1), and an amino acid sequence of the light chain variable region of the anti-CD73 antibody is set forth in SEQ ID NO: 4 (preferably, a nucleic acid sequence is set forth in SEQ ID NO: 3);
an amino acid sequence of the heavy chain variable region of the anti-CD73 antibody is set forth in SEQ ID NO: 6 (preferably, a nucleic acid sequence is set forth in SEQ ID NO: 5), and an amino acid sequence of the light chain variable region of the anti-CD73 antibody is set forth in SEQ ID NO: 8 (preferably, a nucleic acid sequence is set forth in SEQ ID NO: 7);
an amino acid sequence of the heavy chain variable region of the anti-CD73 antibody is set forth in SEQ ID NO: 10 (preferably, a nucleic acid sequence is set forth in SEQ ID NO: 9), and an amino acid sequence of the light chain variable region of the anti-CD73 antibody is set forth in SEQ ID NO: 12 (preferably, a nucleic acid sequence is set forth in SEQ ID NO: 11); or
an amino acid sequence of the heavy chain variable region of the anti-CD73 antibody is set forth in SEQ ID NO: 10 (preferably, a nucleic acid sequence is set forth in SEQ ID NO: 9), and an amino acid sequence of the light chain variable region of the anti-CD73 antibody is set forth in SEQ ID NO: 14 (preferably, a nucleic acid sequence is set forth in SEQ ID NO: 13);
preferably, wherein a heavy chain constant region of the anti-CD73 antibody is Ig gamma-1 chain C region, ACCESSION: P01857; and a light chain constant region is Ig kappa chain C region, ACCESSION: P01834, and more preferably, the heavy chain constant region of the anti-CD73 antibody has the following mutations based on the sequence set forth in ACCESSION: P01857 according to an EU numbering system:
L234A and L235A; or
L234A and G237A; or
L235A and G237A;
or
L234A, L235A and G237A
or one or more mutations selected from:
N297A, D265A, D270A, P238D, L328E, E233D, H268D, P271G, A330R, C226S, C229S, E233P, P331S, S267E, L328F, A330L, M252Y, S254T, T256E, N297Q, P238S, P238A, A327Q, A327G, P329A, K322A, T394D, G236R, G236A, L328R, A330S, P331S, H268A, E318A and K320A; and
most preferably, an amino acid sequence of the heavy chain constant region of the anti-CD73 antibody is set forth in SEQ ID NO: 21, and an amino acid sequence of the light chain constant region of the anti-CD73 antibody is set forth in SEQ ID NO: 22;
preferably, wherein the anti-CD73 antibody is a monoclonal antibody, a humanized antibody, a chimeric antibody or a multispecific antibody (e.g., a bispecific antibody), and
more preferably, the anti-CD73 antibody is an antibody produced by a hybridoma cell line LT014 deposited at China Center for Type Culture Collection (CCTCC) with the accession number CCTCC NO: C2018137.

3. The pharmaceutical composition according to claim 1, wherein an amino acid sequence of the heavy chain variable region of the immunoglobulin is set forth in SEQ ID NO: 27, and an amino acid sequence of the light chain variable region of the immunoglobulin is set forth in SEQ ID NO: 29; and an amino acid sequence of the heavy chain variable region of the single chain antibody is set forth in SEQ ID NO: 37, and an amino acid sequence of the light chain variable region of the single chain antibody is set forth in SEQ ID NO: 39; or an amino acid sequence of the heavy chain variable region of the immunoglobulin is set forth in SEQ ID NO: 37, and an amino acid sequence of the light chain variable region of the immunoglobulin is set forth in SEQ ID NO: 39; and an amino acid sequence of the heavy chain variable region of the single chain antibody is set forth in SEQ ID NO: 27, and an amino acid sequence of the light chain variable region of the single chain antibody is set forth in SEQ ID NO: 29;
preferably, for the bispecific antibody, wherein, according to an EU numbering system, a heavy chain constant region of the immunoglobulin has the following mutations:
L234A and L235A; or
L234A and G237A; or
L235A and G237A;
or
L234A, L235A, G237A, and more preferably, for the bispecific antibody, wherein, according to the EU numbering system, the heavy chain constant region of the immunoglobulin has or further has one or more mutations selected from:
N297A, D265A, D270A, P238D, L328E, E233D, H268D, P271G, A330R, C226S, C229S, E233P, P331S, S267E, L328F, A330L, M252Y, S254T, T256E, N297Q, P238S, P238A, A327Q, A327G, P329A, K322A, T394D, G236R, G236A, L328R, A330S, P331S, H268A, E318A and K320A,
preferably, the immunoglobulin is of human IgG1 subtype,
preferably, the anti-PD-1-anti-VEGFA bispecific antibody has a heavy chain amino acid sequence set forth in SEQ ID NO: 23, and a light chain amino acid sequence set forth in SEQ ID NO: 25,
preferably, a heavy chain of the anti-PD-1-anti-VEGFA bispecific antibody is encoded by a nucleotide sequence set forth in SEQ ID NO: 24, and a light chain thereof is encoded by an amino acid sequence set forth in SEQ ID NO: 26,
preferably, in the anti-PD-1-anti-VEGFA bispecific antibody, the single chain antibody is linked to the C terminus of the heavy chain of the immunoglobulin, preferably, one immunoglobulin molecule is linked to two single chain antibody molecules, and more preferably, the two single chain antibody molecules are identical,
preferably, in the anti-PD-1-anti-VEGFA bispecific antibody, two single chain antibodies are present, and one terminus of each single chain antibody is linked to the C terminus or the N terminus of one of the two heavy chains of the immunoglobulin, more preferably, the single chain antibody is linked to the C terminus of the heavy chain of the immunoglobulin, preferably, in the anti-PD-1-anti-VEGFA bispecific antibody, the first protein functional region is linked to the second protein functional region either directly or via a linker fragment; and/or the heavy chain variable region of the single chain antibody is linked to the light chain variable region of the single chain antibody either directly or via a linker fragment,
preferably, the linker fragment is (GGGGS)n, wherein n is a positive integer, preferably, n is 1, 2, 3, 4, 5 or 6,
preferably, the first protein functional region is linked to the second protein functional region via a first linker fragment; and the heavy chain variable region of the single chain antibody is linked to the light chain variable region of the single chain antibody via a second linker fragment; the first linker fragment and the second linker fragment are the same or different;
preferably, amino acid sequences of the first linker fragment and second linker fragment are independently selected from SEQ ID NO: 47 and SEQ ID NO: 48;
preferably, amino acid sequences of the first linker fragment and second linker fragment are set forth in SEQ ID NO: 48,
preferably, in the bispecific antibody, the numbers of the first protein functional region and the second protein functional region are each independently 1, 2 or more, and
preferably, the anti-PD-1-anti-VEGFA bispecific antibody is a monoclonal antibody or a humanized antibody.

4. The pharmaceutical composition according to any one of claims 1-3, wherein the antigen-binding fragment is selected from Fab, Fab', F(ab')₂, Fd, Fv, dAb, Fab/c, a complementarity determining region fragment, a single chain antibody (e.g., scFv), a humanized antibody, a chimeric antibody and a bispecific antibody.

5. A combination product (e.g., a kit) comprising a first product and a second product in separate packages, wherein,
the first product comprises the anti-CD73 antibody or the antigen-binding fragment thereof as defined in any one of claims 1-3;
the second product comprises the anti-PD-1-anti-VEGFA bispecific antibody as defined in any one of claims 1-3;
preferably, the combination product further comprises a third product in a separate package comprising one or more chemotherapeutics,
preferably, the first product and the second product further independently comprise one or more pharmaceutically acceptable auxiliary materials;
preferably, the combination product further comprises a product instruction, and preferably, the instruction states that the unit dose of the anti-CD73 antibody and/or the anti-PD-1-anti-VEGFA bispecific antibody as defined in any one of claims 1-3 is 0.1-100 mg, preferably 1-10 mg per kg body weight; alternatively, the unit dose of the anti-CD73 antibody and/or the anti-PD-1-anti-VEGFA bispecific antibody as defined in any one of claims 1-3 is 10-1000 mg, preferably 50-500 mg, 100-400 mg, 150-300 mg, 150-250 mg or 200 mg in each subject, and preferably the instruction states that the anti-CD73 antibody and/or the anti-PD-1-anti-VEGFA bispecific antibody is administered twice a day to about once every other day, or once every 3 days, 4 days, 5 days, 6 days, 10 days, 1 week, 2 weeks, 3 weeks, 4 weeks, 5 weeks or 6 weeks,
preferably, in the combination product, the mass ratio of the anti-CD73 antibody or the antigen-binding fragment thereof to the anti-PD-1-anti-VEGFA bispecific antibody is (1:5)-(5:1), e.g., 1:5, 1:4, 1:3, 1:2, 1:1, 2:1, 3:1, 4:1 or 5:1, based on the mass of the antibody, and
preferably, wherein the anti-CD73 antibody, the anti-PD-1-anti-VEGFA bispecific antibody and/or the chemotherapeutic drug is in a form suitable for administration by subcutaneous injection, intradermal injection, intravenous injection, intramuscular injection or intralesional injection, preferably in a form suitable for administration by intravenous injection or intravenous drip infusion, and preferably in a liquid form.

6. A method for treating and/or preventing a tumor, comprising administering to a patient a therapeutically effective amount of drug A and a therapeutically effective amount of drug B, wherein the drug A comprises the anti-CD73 antibody or the antigen-binding fragment thereof as defined in any one of claims 1-3, and the drug B comprises the anti-PD-1-anti-VEGFA bispecific antibody as defined in any one of claims 1-3, preferably the drug A and the drug B are administered either simultaneously or sequentially, wherein the sequential administration is that the drug A is administrated firstly or the drug B is administrated firstly,
preferably, the method further comprises administering in combination with one or more chemotherapeutic drugs (preferably the chemotherapeutic drug is a chemotherapeutic agent or a growth inhibitor, a targeted therapeutic agent (e.g., an antibody-drug conjugate, an antibody or an antigen-binding fragment thereof), a T cell expressing a chimeric antigen receptor, an angiogenesis inhibitor, an antineoplastic agent, a cancer vaccine, an adjuvant and a combination thereof, an alkylating agent, an antimetabolite, an antibiotic, a plant-based and/or hormonal drug, preferably cyclophosphamide, pemetrexed, a platinum-based drug such as cisplatin, carboplatin, oxaliplatin, adriamycin, paclitaxel, vinca alkaloid, tamoxifen, megestrol, goserelin, asparaginase and/or a fluorouracil antineoplastic drug), preferably the anti-CD73 antibody, the anti-PD-1-anti-VEGFA bispecific antibody and the chemotherapeutic drug are administered either simultaneously or sequentially, and more preferably, the anti-CD73 antibody and the anti-PD-1-anti-VEGFA bispecific antibody are administered before or after surgical treatment, and/or before or after radiotherapy,
preferably, the chemotherapeutic agent or the growth inhibitor is selected from an alkylating agent, an anthracycline, an anti-hormonal agent (such as an anti-androgen agent), an aromatase inhibitor, a protein kinase inhibitor (such as a tyrosine kinase inhibitor), a lipid kinase inhibitor, an antisense oligonucleotide, a ribozyme, an anti-metabolite, a topoisomerase inhibitor, a cytotoxic agent or an anti-tumor antibiotic, a proteasome inhibitor, an anti-microtubule agent, an EGFR antagonist, a VEGF antagonist, a PD-1 antagonist, an angiopoietin 2 antagonist, a retinoid, a histone deacetylase inhibitor, and a combination thereof,
preferably, the targeted therapeutic agent is selected from a B-raf inhibitor, an MEK inhibitor, a K-ras inhibitor, a c-Met inhibitor, an Alk inhibitor, a phosphatidylinositol 3-kinase inhibitor, an Akt inhibitor, an mTOR inhibitor, a VEGF inhibitor, a CD73 inhibitor, a PARP inhibitor, a PD-1 inhibitor, a diphosphatidylglycol 3-kinase/mTOR inhibitor, and a combination thereof,
preferably, the antibody-drug conjugate comprises a drug selected from the group consisting of: maytansine, monomethyl auristatin E, calicheamicin, esperamicin, and a radioisotope chelating agent,
preferably, the CD73 inhibitor includes, but is not limited to, one or more of BMS-986179, MEDI9447, NZV930, CPI-006, AB680, LY-3475070, TJ004309[3], ORIC-533, IPH5301AB680 and LY-3475070, and
preferably, the PARP inhibitor is selected from one or more of etoposide, olaparib, rucaparib, niraparib, talazoparib, fluzoparib, veliparib ER, ABT-472, ABT-767, Stenoparib, AST-6828, AG-PD, ANG-2864, ANG-3038, ANG-3186, AZD-5305, AZ-0108, AZD-2461, AMXI-5001, AMXI-2001, AMXI-3001, AMXI-7001, AMXI-9001, pamiparib, ZYTP-1, CK-102, XZ-120312, YHP-743, iobenguane I 131, rucaparib camsylate, CVL-218, CPH-101, CPH-102, CBX-11, CBX-15, minocycline, DB-207, DPS-102, E-7016, iobenguane I 131, MK-2512, HCX-014, HWH-340, IDX-1197, IDX-1197, senaparib, IMP-04100, IMP-04111, IMP-04149, IMP-04249, IMP-04307, IMP-04356, JPI-289, JPI-547, JPI-283, fluzoparib, GT-1620, iobenguane I 131, DR-2313, MP-124, H-10, NT-125, BGP-15, NMSP-293, NMSP-293, NMSP-118, NMSP-648, NMSP-914, DB-207, NUV-1156, NUV-1176, JPI-289, Stenoparib, OX-401, NU-1025, NU-1085, PLX-376, R-554, RBN-2397, RBN-012759, PJ-34, INO-1001, WW-46, BSI-401, iniparib, SOMCL-9112, SC-10914, HTMC-0435, SRX-3128, TSL-1502, PJ-34, CEP-8983, CK-102, THG-009, talazoparib SR, L-2286, mitoparib and WB-1340.

7. A unit formulation, preferably used for treating a tumor, and comprising 1-10000 mg (preferably 10-1000 mg, preferably 50-500 mg, 100-400 mg, 150-300 mg, 150-250 mg or 200 mg) of the anti-CD73 antibody as defined in any one of claims 1-3 and 1-10000 mg (preferably 1-1000 mg, preferably 50-500 mg, 100-400 mg, 150-300 mg, 150-250 mg, 200 mg or 100 mg) of the anti-PD-1-anti-VEGFA bispecific antibody as defined in any one of claims 1-3, and optionally one or more of the chemotherapeutic drugs (such as a platinum-based drug and/or a fluorouracil antineoplastic drug) as defined in claim 6; wherein the anti-CD73 antibody, the anti-PD-1-anti-VEGFA bispecific antibody and the chemotherapeutic drug are packaged separately,
preferably, the unit dose of the anti-CD73 antibody and/or the anti-PD-1-anti-VEGFA bispecific antibody as defined in any one of claims 1-3 is 0.1-100 mg, preferably 1-10 mg per kg body weight; alternatively, the unit dose of the anti-CD73 antibody and/or the anti-PD-1-anti-VEGFA bispecific antibody as defined in any one of claims 1-3 is 10-1000 mg, preferably 50-500 mg, 100-400 mg, 150-300 mg, 150-250 mg or 200 mg in each subject, and
preferably, the dose is given from twice daily to about once every other day, or once every 3 days, 4 days, 5 days, 6 days, 10 days, 1 week, 2 weeks, 3 weeks, 4 weeks, 5 weeks or 6 weeks.

8. A single dose unit, preferably used for treating a tumor, and comprising 0.1-10000 mg (preferably 1-1000 mg, preferably 50-500 mg, 100-400 mg, 150-300 mg, 150-250 mg, 200 mg or 100 mg) of the anti-CD73 antibody as defined in any one of claims 1-3 and 0.1-10000 mg (preferably 1-1000 mg, preferably 50-500 mg, 100-400 mg, 150-300 mg, 150-250 mg, 200 mg or 100 mg) of the anti-PD-1-anti-VEGFA bispecific antibody as defined in any one of claims 1-3.

9. The method according to claim 6, the unit formulation according to claim 7, or the single dose unit according to claim 8, wherein the tumor is selected from one or more of the following:
cervical cancer (e.g., metastatic cervical cancer), endometrial cancer, lung cancer such as small cell lung cancer and non-small cell lung cancer (e.g., squamous non-small cell lung cancer or non-squamous non-small cell lung cancer), throat cancer, esophageal cancer, esophageal squamous cancer, thyroid cancer, mesothelioma, gastrointestinal cancer such as gastric cancer ((including micro satellite stability (MSS) and mismatch repair dysfunction/microsatellite high instability (dMMR/MSI-H) type), e.g., advanced gastric cancer, gastric adenocarcinoma or gastroesophageal junction adenocarcinoma), and intestinal cancer (e.g., rectal cancer, colon cancer, colorectal cancer (including micro satellite stability (MSS) and mismatch repair dysfunction/microsatellite high instability (dMMR/MSI-H) type)), liver cancer (e.g., hepatocellular carcinoma, hepatobiliary cancer), biliary tract cancer (e.g., cholangiocarcinoma and gallbladder cancer), pancreatic cancer, pancreas cancer, renal cancer, ovarian cancer (e.g., advanced ovarian cancer), fallopian tube cancer, anal epidermoid carcinoma, peritoneal cancer, glioma, neuroglioma, recurrent glioma, skin cancer, melanoma, hematological malignancy (such as leukemia (e.g., acute myeloid leukemia)), lymphoma (e.g., Hodgkin's lymphoma, non-Hodgkin's lymphoma), multiple myeloma, B-lymphoma (e.g., plasma cell carcinoma), bone cancer, sarcoma (e.g., leiomyosarcoma, rhabdomyosarcoma), osteosarcoma, chondrosarcoma, neuroblastoma, myeloma (e.g., multiple myeloma), large cell neuroendocrine cancer, urothelial carcinoma (e.g., upper urothelial carcinoma or bladder cancer), prostate cancer (including metastatic castration-resistant prostate cancer (mCRPC)), testicular cancer, triple-negative breast cancer, peripheral T-cell lymphoma, nasopharyngeal cancer, microsatellite high instability (MSI-H) or mismatch repair dysfunction (dMMR) type solid tumor, head and neck cancer, brain cancer (e.g., aggressive brain cancer), squamous cell carcinoma, basal cell carcinoma, adenoma (e.g., breast cancer, thymus cancer, ileocecal adenocarcinoma, ampullate adenocarcinoma, pancreatic ductal adenocarcinoma, mucinous or serous cystadenocarcinoma), chorionic epithelioma, malignant hydatidiform mole, malignant sertoli cell-stromal cell tumor, malignant granulocytoma, dysgerminoma, glioblastoma, mycosis, Merkel cell carcinoma, intrahepatic bile duct carcinoma, Merkel cell carcinoma, squamous cell anorectal cancer, squamous cell carcinoma of the tongue, squamous cell carcinoma of the head and neck, and other hematological malignant tumors.
